# EUROPEAN PATENT APPLICATION

(11) **EP 4 154 889 A1**
(43) Date of publication of application: **29.03.2023**
(21) Application number: 22197344.9
(22) Date of filing: 23.09.2022
(51) Int. Cl.: A61K 31/4985, A61K 31/519, A61K 31/53, A61K 45/06, A61P 25/02, A61P 21/00, A61P 27/02, A61P 25/28, A61P 25/08

(54) **PDE5 INHIBITOR FOR USE IN THE TREATMENT OF MEDICAL CONDITIONS ASSOCIATED WITH MITOCHONDRIAL COMPLEX V DEFICIENY**

(30) Priority: 24.09.2021 EP 21198827
(71) Applicant: Charité - Universitätsmedizin Berlin, 10117 Berlin (DE); Max-Delbrück-Centrum für Molekulare Medizin in der Helmholtz-Gemeinschaft, 13125 Berlin (DE)
(72) Inventor: Schülke-Gerstenfeld, Markus, 10555 Berlin (DE); Prigione, Alessandro, 40225 Düsseldorf (DE)
(74) Representative: Hertin und Partner Rechts- und Patentanwälte PartG mbB

(57) **Abstract**

The invention relates to a PDE5 inhibitor for use in the treatment and/or prevention of a medical condition associated with mitochondrial Complex V (ATP synthase) deficiency in a human subject. In embodiments, the mitochondrial Complex V deficiency comprises at least one mutation within a structural subunit gene, or within an assembly gene, of the mitochondrial Complex V (ATP synthase). In embodiments, the invention relates to PDE5 inhibitors, such as sildenafil or tadalafil, in the treatment and/or prevention of a Maternally Inherited Leigh syndrome (MILS), a Neuropathy, an Ataxia or a Retinitis Pigmentosa (NARP) syndrome, or a neurological syndrome associated with a mitochondrial Complex V deficiency. In embodiments, the invention relates to treatment of mitochondrial Complex V deficiency caused by at least one mutation in nuclear or mitochondrial DNA, preferably DNA mutations in the MT-ATP6 gene causing MILS or NARP. The invention further relates to a pharmaceutical composition comprising a PDE5 inhibitor for use in the treatment and/or prevention of a medical condition associated with mitochondrial Complex V deficiency.

## Description

The invention relates to the field of pharmaceutical compositions, combinations, and the treatment of medical conditions.

The invention relates to a phosphodiesterase 5 (PDE5) inhibitor for use in the treatment and/or prevention of a medical condition associated with mitochondrial Complex V (ATP synthase) deficiency in a human subject. In preferred embodiments, the mitochondrial Complex V deficiency comprises at least one mutation within a structural subunit gene, or within an assembly gene, of the mitochondrial Complex V (ATP synthase).

In embodiments, the invention relates to PDE5 inhibitors, such as sildenafil or tadalafil, in the treatment and/or prevention of a Maternally Inherited Leigh syndrome (MILS), a Neuropathy, an Ataxia or a Retinitis Pigmentosa (NARP) syndrome, or a neurological syndrome associated with a mitochondrial Complex V deficiency. In embodiments, the invention relates to treatment of mitochondrial Complex V deficiency caused by at least one mutation in nuclear or mitochondrial DNA, preferably DNA mutations in the MT-ATP6 gene causing MILS or NARP. The invention further relates to a pharmaceutical composition comprising a PDE5 inhibitor for use in the treatment and/or prevention of a medical condition associated with mitochondrial Complex V deficiency.

### BACKGROUND OF THE INVENTION

Mitochondrial Complex V is essential for energy production in the form of ATP and is the last of five mitochondrial complexes for the multistep process of oxidative phosphorylation. Mitochondrial Complex V deficiency includes a lack of a Complex V protein, a loss of a Complex V protein, its limited function, or a complete loss of its function. Mitochondrial Complex V deficiency can cause a variety of signs and symptoms that affect many organs and systems of the body, especially the nervous system and heart. Such disorders can be life-threatening in both infancy and later life and often leads to premature death in patients. Mitochondrial Complex V related diseases comprise Leigh Syndrome, Leigh-like syndrome, Maternally Inherited Leigh Syndrome, and Neuropathy, Ataxia, and Retinitis Pigmentosa syndrome. [1]

Leigh syndrome is a rare (incidence <1:2,000) and a severe developmental disorder of the nervous system. It can be based on numerous genetic defects, which can be located either on nuclear DNA (inherited according to Mendelian rules) or on mitochondrial DNA (mtDNA, maternal inheritance only). A mitochondrially caused variant is therefore designated as "Maternally Inherited Leigh Syndrome" (MILS) or Neuropathy, Ataxia, and Retinitis Pigmentosa (NARP) syndrome. Numerous mitochondrial genetic defects have been described so far. About 30% of patients with MILS-typical Leigh syndrome have a mutation within the MT-ATP6 gene located on the mtDNA which occurs in more than 90% of mitochondrial DNA copies per cell. The NARP syndrome is also caused by mutations within the MT-ATP6 gene but in only about 80% of mitochondrial DNA copies per cell or less. The MT-ATP6 gene encodes a structural subunit of F1F0-ATPase, the enzyme complex that synthesizes adenosine triphosphate (ATP) in the mitochondrion, the body's universal source of energy. Because each cell contains numerous copies of mtDNA, a certain percentage of the mtDNA copies must be mutated (mutation load) before clinical symptoms occur. [1-3]

ATP deficiency results in impaired function, especially of organs that have a high energy demand, i.e. the nervous system, muscles, liver, but also endocrine glands and sensory organs. Common to all subtypes is selective damage to dopaminergic neurons in the brainstem and basal ganglia, which may be particularly apparent in the context of metabolic crises.

Patients with mitochondrial Complex V deficiency related diseases, such as the Leigh syndrome, often die in early childhood, commonly from heart or respiratory failure. Some patients with a less severe course or late manifestation also reach teenage or early adulthood.

Treatments of mitochondrial disorders have been suggested as combination therapies or specifically for disorders affecting a particular organ, such as ocular disorders, or for treatment targeting only a particular part or organ, but not for disorders or syndromes caused by mitochondrial gene mutations. [5-8] However, studies more often report on missing links and the present poor understanding on the role of mitochondrial proteins in the pathogenesis of certain disorders and the lack of suitable treatment options, rather than successfully applied treatments for mitochondriopathies. [9] Sildenafil was used in patients with a TMEM70 mutation causing a nuclear ATP synthase deficiency for treating pulmonary hypertension. [9] Furthermore, mitochondrial defects and ATP synthesis were not ameliorated by treatment with a PDE5 inhibitor in a mouse model for a mitochondrial disorder (Duchenne muscular dystrophy). [12]

Mitochondriopathies are among the most difficult diseases to study and treat. The cell types affected are difficult or impossible to access for the development of a therapy. In many cases, there are no suitable animal models, as in the case of diseases caused by mutations in mitochondrial genes. Moreover, mitochondrial diseases usually affect a large number of organs, are heterogeneous in expression, and are in many cases systemic, so that research into potential therapies is significantly hampered. Despite some initial promising results, it has not yet been possible to treat groups of patients with mitochondrial complex V (ATP synthase) deficiency, such as Leigh syndrome. The results from various studies often come from mouse experiments, which either cannot be applied in humans at all or have been shown to be ineffective in humans. [7, 8, 10-12]

Currently available treatments include vitamin B, coenzyme Q or L-carnitine, and oral sodium bicarbonate or sodium citrate to treat lactic acidosis.[4] These treatments are poorly effective and the prognosis for these patients is extremely poor. None of these treatments may reverse existing and progressed symptoms. Gene therapy measures are not accessible especially for patients with mutations in mitochondrial DNA due to the high copy number of mitochondrial DNA per cell with occasional variable percentage of mutations distributed among mitochondrial DNA copies. To date, there are no therapeutic options for Leigh syndrome, MILS, NARP and Leigh-like syndrome that influence the pathophysiological process.

Thus, there is a critical and currently unmet need for effective and/or symptom-reversing treatments for mitochondrial Complex V deficiency-associated diseases, including MILS, NARP, Leigh syndrome, and Leigh-like syndrome.

### SUMMARY OF THE INVENTION

In light of the prior art, the technical problem underlying the present invention is to provide alternative or improved means for the treatment and/or prevention of a medical condition associated with mitochondrial Complex V deficiency.

The technical problem may also be viewed as the provision of means for the treatment and/or reduction of risk and/or reversing symptoms and/or delaying progress of symptoms of medical conditions associated with a mitochondrial Complex V deficiency, more particular MILS, NARP, Leigh syndrome, and Leigh-like syndrome.

The technical problem may also be viewed as the provision of means for the prevention and/or treatment of mitochondrial Complex V deficiency associated increase of mitochondrial membrane potential, decrease or increase of intracellular calcium signaling and/or decrease of mitochondrial energy supply in the cell of a human subject.

The technical problem may further be viewed as the provision of means for the prevention and/or treatment and/or reversion of mitochondrial Complex V deficiency-associated muscular hypotonia, hypertrophic cardiomyopathy, psychomotor delay, encephalopathy, peripheral neuropathy, lactic acidosis, brain stem and basal ganglia degeneration, hepatomegaly, and palliative care.

These problems are solved by the features of the independent claims. Preferred embodiments of the present invention are provided by the dependent claims.

The invention therefore relates to a phosphodiesterase 5 (PDE5) inhibitor for use in the treatment and/or prevention of a medical condition associated with mitochondrial Complex V (ATP synthase) deficiency in a human subject.

In embodiments, the invention therefore relates to a phosphodiesterase 5 (PDE5) inhibitor for use in the treatment and/or prevention of a medical condition caused by mitochondrial Complex V (ATP synthase) deficiency in a human subject.

The invention also relates to a method for treating, preventing and/or reducing the risk of a medical condition associated with mitochondrial Complex V (ATP synthase) deficiency in a human subject comprising administering a PDE5 inhibitor to said subject.

The invention also relates to a method for treating, preventing and/or reducing the risk of a medical condition caused by mitochondrial Complex V (ATP synthase) deficiency in a human subject comprising administering a PDE5 inhibitor to said subject.

References to medical conditions associated with mitochondrial Complex V (ATP synthase) deficiency also include medical condition caused by mitochondrial Complex V (ATP synthase) deficiency, and vice versa.

In embodiments, mitochondrial Complex V deficiency-associated diseases (or related diseases, or diseases caused by mitochondrial Complex V deficiency) are commonly caused by or comprise at least one DNA mutation in a structural subunit gene, or in an assembly gene, of the mitochondrial Complex V (ATP synthase).

The present invention provides means and methods for the use of PDE5 inhibitors for treatment of mitochondrial Complex V deficiency and related diseases in a human subject. In one embodiment, for example, a method of the present invention is described for treating mitochondrial Complex V deficiency-associated diseases in a human subject, comprising treating the patient with an effective amount of PDE5 inhibitor, or a pharmaceutically acceptable salt, derivative, or composition thereof.

In one embodiment, the human subject is a patient suffering from the mitochondrial Complex V deficiency-related disease MILS.

In one embodiment, the human subject is a patient suffering from the mitochondrial Complex V deficiency-related disease NARP.

In one embodiment, the human subject is a patient suffering from the mitochondrial Complex V deficiency-related disease Leigh-Syndrome.

In one embodiment, the human subject is a patient suffering from the mitochondrial Complex V deficiency-related disease Leigh-like syndrome.

In some embodiments, the present invention further provides means and methods for the use of PDE5 inhibitors for treating, preventing, reducing and/or reversing symptoms of medical conditions associated with mitochondrial Complex V deficiency, wherein said symptoms comprise paralytic events, motor and sensory neuropathy, metabolic crises events, lactic acidosis, cardiomyopathy, seizures, encephalopathy, stroke-like episodes, endocrine abnormalities, anemia, exercise insufficiency, muscle weakness, loss of muscle tone and/or muscular hypotonia, respiratory insufficiency, retinitis pigmentosa, ataxia, increased susceptibility to infections, developmental delay, intellectual disability in a human subject compared to control.

In a preferred embodiment, the PDE5 inhibitor for use as a medicament as described herein, is characterized in that the treatment of a medical condition associated with a mitochondrial Complex V deficiency comprises the treatment of a subject with detectable mutation in target cells compared to a control, such as healthy controls.

In the prior art, PDE5 inhibitors, such as sildenafil, have been suggested as secondary therapeutics for treating pulmonary hypertension in patients suffering from various disease backgrounds, but have not been suggested for the treatment of muscular and neurological symptoms in a patient with complex V deficiency as a primary therapy. Therefore, the PDE5 inhibitor can be beneficially applied for all neurological and muscular symptoms associated with complex V deficiency in the patient groups described herein. In particular, the invention discloses the PDE5 inhibitor for use as a single therapy. Research has been ongoing and there is at present no current treatment for patients suffering from MILS, NARP, Leigh Syndrome or Leigh Like Syndrome or patients with mutations in the MT-ATP6 or MT-ATP8 gene. The therapeutic efficacy demonstrated herein in human subjects represents a significant advance in treatment options for the relevant medical conditions, and could not have been expected as such from the obvious difficulties in research and treatment evident in the field.

In one embodiment, for example, a method of the present invention for the use of PDE5 inhibitors is described for treating, preventing and/or reducing the risk of said subject for metabolic crises, palliative care or mortality, or resolves the need of the subject to receive palliative care.

The present invention provides means having a surprisingly beneficial effect on patient survival in mitochondrial Complex V deficiency associated mortality and risk for receiving palliative care.

As evident from the experimental support provided (refer Examples), mitochondrial Complex V deficiency causes significantly increased mitochondrial membrane potential and disturbed intracellular calcium levels in neuronal progenitor cells from patients suffering from MILS. Treatment with a PDE5 inhibitor, including tadalafil and sildenafil, reduced mitochondrial membrane potential and intracellular calcium disturbance to levels comparable to healthy controls. PDE5 inhibitors prevent cGMP degradation and neither corrects the mutation nor directly interferes with the mitochondrial Complex V. The experimental results obtained in vitro are surprising and could not have been expected by a skilled person from prior art.

In one embodiment, said treatment prevents patient suffering from mitochondrial Complex V deficiency-related disease from palliative care and reverses existing symptoms, such as exercise insufficiency, muscle weakness, muscular hypotonia, respiratory insufficiency, and developmental delay.

In the state of the art, PDE5 inhibitors have been known for more than 30 years and had not previously been applied successfully in the relevant patient groups (Leigh syndrome, Leigh Like Syndrome, MILS, NARP). The treatment results described herein are also surprising, in that the disease was at an advanced stage of progression in treated patients, and affected many organs, including essential organs, and some of the patients were already intended for palliative care (refer to Example 4). The skilled person knows from the state of the art that a patient in such an advanced stage usually no longer responds to treatment and a reversibility of symptoms would typically not be expected. The skilled person could not have predicted with any certainty that treatment with a PDE5 inhibitor would achieve such positive therapeutic effects in patients as described in the examples, including stopping disease progression, withdrawal from palliative care, reversibility of cardiological changes, return of patient mobility and independence, and that treatment success would be achieved at different stages of the disease.

As further being evident from individual therapy trials (refer Examples), patients suffering from MILS have been successfully treated with the PDE5 inhibitor sildenafil. The treatment shows tremendous and unexpected therapeutic success including improvement and reversion of symptoms and episodes of MILS. For example, patient A, a teenage male and suffering from MILS, presented with encephalopathy, cerebral seizures, severe leg-emphasized motor-sensory neuropathy, cardiomyopathy with chronic myocardial damage. He was in need of PEG tube feeding and mechanical ventilation though a tracheostoma due to respiratory insufficiency. Palliative care was considered for MILS patient A. Unexpectedly, after treatment with sildenafil was initiated, patient A regained the abilities to breathe and eat independently, the tracheostoma could be closed, he was able to lift his arms and legs against gravity and was able to leave the hospital and start attending school. The cardiomyopathy has reversed, and cardiac control investigations were normal. After about a year, patient A continues to regain motor abilities and can even lift himself into a wheelchair and walk intermittently with walkers with forearm supports. Cerebral seizures did not reoccur. After three years of treatment his state of health is stable and he did not suffer from any metabolic crisis ever since.

Another patient C suffering from MILS, 9 months old and male, exhibited severe muscle hypotonia, hypertrophic cardiomyopathy, and developmental delay and was too weak to suckle. During his 9 months of life he had to be admitted 10 times to hospital for metabolic crises. Palliative care was considered for MILS patient C. Soon after the treatment of patient C with PDE5 inhibitor sildenafil was initiated, no palliative care was required and no further metabolic crisis occurred. Patient C progressed in motor development, including turning, independent swallowing, sitting, and head control. After 3 years of treatment his clinical state is stable. These remarkable and surprising findings could not have been expected by a skilled person nor have they been shown in the prior art.

In all individual therapy trials provided in the Examples, sildenafil was found generally well tolerated and with a safety profile acceptable to patients, even for long term use. Treatment, reversion, or prevention, as used herein, is most advantageous for efficacy, gentle on the patient, and is also associated with fewer side effects, if any. Surprisingly, the treatment was successful regardless of age. A person skilled in the art would not have expected from the prior art that the treatment could be used equally successfully in infants and young adults, as shown in the examples. Treatment with a PDE5 inhibitor, as disclosed herein, is thus independent of patient age and can be used at any stage of the disease.

In some embodiments, the treatment is administered to patients at any age.

In some embodiments, the subject is or has been treated additionally with one or more therapeutic drugs, i.e. biotin, riboflavin and/or CoQ10.

In some embodiments, the invention relates to a PDE5 inhibitor for use in the treatment and/or prevention of a medical condition associated with mitochondrial Complex V deficiency, wherein said medical condition is a mitochondrial Complex V deficiency-related disease.

In some embodiments, the invention relates to a PDE5 inhibitor for use in the treatment and/or prevention of medical condition associated with mitochondrial Complex V deficiency, wherein the condition comprises or is caused by at least one DNA mutation within a mitochondrial Complex V structural subunit gene or within an assembly gene of a mitochondrial Complex V, wherein said gene is located on chromosomal DNA in the nucleus of the human subject.

In some embodiments, the invention relates to a PDE5 inhibitor for use in the treatment and/or prevention of medical condition associated with mitochondrial Complex V deficiency, wherein the condition comprises or is caused by at least one DNA mutation within a mitochondrial Complex V structural subunit gene or within an assembly gene of a mitochondrial Complex V, wherein said gene is located extra chromosomally on mitochondrial DNA in the mitochondria of the human subject.

In one embodiment, the mitochondrial Complex V deficiency related disease is selected from MILS, NARP, Leigh syndrome, and Leigh like syndrome, preferably MILS and NARP.

In some embodiments, the treatment is administered to patients at any disease stage of Leigh Syndrome, Leigh Like Syndrome, MILS and NARP.

In one embodiment, the PDE5 inhibitor is used in the treatment and/or prevention of the medical condition associated with mitochondrial Complex V deficiency in the human subject, wherein said human subject suffer from MILS, NARP, Leigh syndrome, or Leigh like syndrome, preferably MILS or NARP.

In one embodiment, the human subject suffering from mitochondrial Complex V deficiency has at least one mutation or two or more mutations in one gene or in at least one gene or in two or more genes, wherein said mutation(s) affect Complex V of the mitochondrial electron transport chain.

Genes that express proteins of the mitochondrial Complex V structural subunits and the assembly proteins of the mitochondrial ATP synthase can be easily selected from public data bases, such as OMIM, malacards.org, NCBI or Orphan.net, by a skilled person.

In some embodiments, the mutation in the mitochondrial Complex V structural subunit or assembly gene comprises a variant at one or more nucleic acid positions is selected from Table 2 in mitochondrial DNA or the nuclear genome.

In one embodiment, the DNA mutation is a mitochondrial gene mutation for a structural subunit or an assembly factor of the mitochondrial Complex V.

In some embodiments, the patient suffering from mitochondrial Complex V deficiency has at least one mutation or two or more mutations in one gene or in at least one gene or in two or more genes, wherein said gene is a mitochondrial gene and can be MT-ATP6 and/or MT-ATP8.

In some embodiments, the DNA mutation occurs in the mitochondrial membrane subunit 6 gene (MT-ATP6).

Patients with mutations in the mitochondrial genes for a structural subunit or an assembly factor of the mitochondrial complex V particularly benefit from treatment with a PDE5 inhibitor, such as sildenafil. In particular, patients with mutations in the MT-ATP6 gene and/or MT-ATP8 gene benefit. In some embodiments, the patient suffering from mitochondrial Complex V deficiency has at least one mutation or two or more mutations in one gene or in at least one gene or in two or more genes, wherein said gene can be selected from a group of ATPAF2, TMEM70, ATP5F1E, ATP5F1A, ATP5F1D, ATP5MD, ATP5MC3, ATP5MK, ATP5PO, MT-ATP6, MC5DM1, MT-ATP8, and/or MC5DM2.ln one embodiment, the patient suffering from Leigh syndrome has at least one mutation or two or more mutations in one gene or in at least one gene or in two or more genes, wherein said mutation(s) affect Complex V of the mitochondrial electron transport chain.

In another embodiment, the patient suffering from Leigh like syndrome has at least one mutation or two or more mutations in a gene or in at least one gene or in at least two genes, wherein said mutation(s) affect Complex V of the mitochondrial electron transport chain.

In one embodiment, the patient suffering from MILS has a mutation or at least one mutation or two or more mutations in one gene or in at least one gene or in two or more genes, wherein said mutation(s) affect Complex V of the mitochondrial electron transport chain.

In one embodiment, the patient suffering from NARP has at least one mutation or two or more mutations in one gene or in at least one gene or in two or more genes, wherein said mutation(s) affect Complex V of the mitochondrial electron transport chain.

In preferred embodiments, the patient suffering from MILS or NARP has at least one mutation or two or more mutations within the mitochondrial MT-ATP6 gene.

In preferred embodiments, the patient suffering from MILS or NARP has at least one mutation or two or more mutations within the mitochondrial MT-ATP8 gene.

As described at length herein, the composition comprising a PDE5 inhibitor, represents a novel approach towards developing an effective measure to treat and/or prevent a medical condition associated with mitochondrial Complex V deficiency, particularly in subjects suffering from MILS. At present, said patients are not appropriately treated and have a high mortality as no effective medication is available for the patient group described herein. As evident from the experimental support provided (refer Examples), decreased mitochondrial membrane potential and disturbed intracellular calcium levels significantly correlates with a medical condition associated with mitochondrial Complex V deficiency, including MILS.

Furthermore, the inventors were already able to demonstrate a surprising and beneficial effect in human subjects that could not have been expected by a skilled person, e.g. therapeutic effect in MILS patients with different mutations, as shown in the Examples. In some cases, the means and treatment as described here were the only and final solution that allowed patients to survive. Most surprisingly, however, treatment with means and methods described herein reversed pre-existing symptoms and associated organ changes, such as cardiomyopathy and developmental delay.

In the prior art, a number of diseases have been disclosed that also involve mitochondrial dysfunction. None of the attempts in preclinical and clinical studies could achieve an improvement in mitochondrial defects or ATP synthesis (eg. NCT01580501). Thus, the skilled person would not have reasonably expected that the treatment of a purely mitochondrial disease would be successful.

In one embodiment, a mitochondrial mutation is heteroplasmic.

In one embodiment, prior to initiating treatment of said subjects, a sample is collected prior to treatment. Said sample is preferably used for screening of mutations in said genes and/or for measuring of the complex V activity, mitochondrial membrane potential and/or metabolic parameters (e.g. lactate and alanine in the serum) described herein in cells, blood, plasma, cerebral fluid or serum according to the standard analytics of the prior art and by means of methods described in the Examples. More details for sample handling and measurement are described in the Examples.

In some embodiments, a target cell for the treatment is a cell carrying at least one mutation causing a medical condition associated with mitochondrial Complex V deficiency.

In embodiment, the mitochondrial gene resulting in a medical condition associated with mitochondrial Complex V deficiency can be MT-ATP6 or MT-ATP8.

In one embodiment, the MT-ATP6 gene mutation refers to any MT-ATP6 gene sequence differing to the reference sequences SEQ ID No: 1 described herein.

In one embodiment, the MT-ATP6 gene mutation is heteroplasmic.

In one embodiment, the MT-ATP8 gene mutation refers to any MT-ATP8 gene sequence differing to the reference sequences SEQ ID No: 1 described herein.

In one embodiment, the MT-ATP8 gene mutation is heteroplasmic.

Heteroplasmic mitochondrial mutations in a mitochondrial gene occur in a specific proportion, i.e. less than 100%, of all mitochondrial gene copies present on mitochondrial DNA copies in a patient cell.

In one embodiment, within the same cell of the patient, the mitochondrial DNA carries gene copies, such as MT-ATP6 gene copies or MT-ATP8 gene copies, that have a mutation as well as gene copies that do not carry a mutation. In one embodiment, the specific proportion of mutated mitochondrial gene copies can be about or at least 1%, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%.

The specific proportion of mutated mitochondrial MT-ATP6 gene copies or MT-ATP8 gene copies can be determined by methods known in the art.

In one embodiment, the PDE5 inhibitor is used for the treatment of a medical condition associated with mitochondrial Complex V deficiency caused by at least one heteroplasmic mutation in mitochondrial DNA.

In one embodiment, a mitochondrial mutation is homoplasmic.

In one embodiment, a cell of a subject has identical copies of mitochondrial DNA at any given locus, in all mitochondria of the cell. For example, homoplasmic MT-ATP6 mutations occur in all cellular copies of the mitochondrial MT-ATP6 gene on the mitochondrial DNA of a subject.

In one embodiment, the MT-ATP6 gene mutation is homoplasmic. In one embodiment, the MT-ATP8 gene mutation is homoplasmic.

In one embodiment, the mitochondrial DNA mutation is homoplasmic or heteroplasmic, wherein said heteroplasmic mitochondrial DNA mutation is present in at least 30% of mitochondria per cell, preferably 60% or more, more preferably 70% or more.

In one embodiment, MILS and NARP are caused by mutations within the MT-ATP6 gene. MILS and NARP differ in the number of mutated mitochondrial DNA copy numbers in a patient cell.

In one embodiment, patients having at least 90% mutated MT-ATP6 copies per cell are classified as MILS patients.

In one embodiment, patients having 80% or less than 80% mutated MT-ATP6 copies per cell are classified as NARP patients.

In one embodiment, the mutation in the mitochondrial Complex V structural subunit or assembly gene comprises a variant at one or more nucleic acid positions selected from Table 3 in mitochondrial DNA or the nuclear genome. As provided in detail below, various mutations are known to be related to mitochondrial Complex V deficiency, and these are provided in table 3.

In one embodiment, the patient suffering from Leigh syndrome, Leigh like syndrome, MILS or NARP has at least one mutation or two or more mutations in one gene or in at least one gene or in two or more genes, wherein said mutation(s) affect mitochondrial Complex V structural or assembly genes. In one embodiment, DNA mutations in genes causing medical condition associated with mitochondrial Complex V deficiency can be identified by genetic screening of patients at risk for disease or presenting symptom of the disease, wherein said genes gene can be selected from a group of ATPAF2, TMEM70, ATP5F1E, ATP5F1A, ATP5F1D, ATP5MD, ATP5MC3, ATP5MK, ATP5PO, MT-ATP6, MC5DM1, MT-ATP8, and/or MC5DM2.

In preferred embodiments, the patient suffering from MILS or NARP has at least one mutation or two or more mutations within the mitochondrial MT-ATP6 gene.

The skilled person is capable of identifying mitochondrial and nuclear genes and mutations within these genes causing the Complex V deficiency and/or Leigh syndrome. For example, publically available databases can be used, such as ClinVar database, HGMD database, OMIM database, and MitoMap for screening and verifying genes and mutations, particularly pathogenic and disease-causing mutations. A mutation can be in frame, out of frame, located within a gene, located downstream of a gene and/or located upstream of a gene.

In one embodiment the DNA mutation is a nuclear mutation and is homozygous or compound heterozygous for a structural subunit or an assembly factor of the mitochondrial Complex V.

In one embodiment, the medical conditions associated with mitochondrial Complex V deficiency can be caused by at least one DNA mutation on nuclear DNA located genes, wherein said genes comprise ATPAF2, TMEM70, ATP5F1E, ATP5F1A, ATP5F1D, ATP5MD, ATP5MC3, ATP5MK, and/or ATP5PO.

In one embodiment, the medical condition comprises a Maternally Inherited Leigh syndrome (MILS), a Neuropathy, an Ataxia or a Retinitis Pigmentosa (NARP) or a neurological syndrome associated with a mitochondrial Complex V deficiency.

In one embodiment, the medical condition comprises MILS (a Maternally Inherited Leigh syndrome), NARP (Neuropathy, an Ataxia or a Retinitis Pigmentosa) or a neurological syndrome associated with a mitochondrial Complex V deficiency.

Without prejudice to the remaining aspects and embodiments of the invention, in some embodiments, medical conditions having a mitochrondrial deficiency that is not caused by mitochondrial genetic defects, preferably is not caused by genetic defects in components of mitochondrial complex V, such as Duchenne muscular dystrophy or Friedreich Ataxia, do not benefit from PDE5 inhibitor treatments as described herein, or do not benefit to the same extent as medical conditions with a mitochondrial genetic defect.

In one embodiment, the invention comprises a method of treating a subject suffering from mitochondrial Complex V deficiency related disease, wherein the subject has a pre-treatment plasma lactate level of greater than or equal to about 2 mmol/liter, greater than or equal to about 3 mmol/liter, greater than or equal to about 4 mmol/liter, or greater than or equal to about 5 mmol/liter. The subject may be treated with PDE5 inhibitor, i.e., sildenafil.

In one embodiment, the invention comprises a method of treating a subject suffering from mitochondrial Complex V deficiency related disease, wherein the subject has a pre-treatment cerebrospinal fluid lactate level of greater than or equal to about 2 mmol/liter, greater than or equal to about 3 mmol/liter, greater than or equal to about 4 mmol/liter, or greater than or equal to about 5 mmol/liter. The subject may be treated with PDE5 inhibitor, i.e., sildenafil.

In one embodiment, the invention comprises a method of treating a subject suffering from Leigh syndrome or Leigh-like syndrome, wherein the subject has a pre-treatment plasma lactate level greater than or equal to about 2 mmol/liter and a cerebrospinal fluid lactate level greater than or equal to about 2 mmol/liter. The subject may be treated with PDE5 inhibitor, i.e. sildenafil.

In one embodiment, the PDE-5 inhibitor is avanafil.

Several PDE5 inhibitors are known in the prior art. In one embodiment, the compound for use in treating mitochondrial Complex V deficiency related disease is selected from the group consisting of sildenafil, avanafil, vardenafil, tadalafil, mirodenafil, udenafil, lodenafil E-8010, Zaprinast, and E-4021.

For all compounds and methods described herein that use a PDE5 inhibitor, a derivative, salt or metabolite thereof may be used, if desired.

For all compounds and methods described herein, the invention also encompasses the use of the compounds described herein for the manufacture of a medicament for use in the treatment of mitochondrial Complex V deficiency-related diseases.

In preferred embodiments, the compounds are administered in concentrations or amounts, or according to dosage regimes, already established in the art, such as those for which regulatory approval has been issued (e.g. by the FDA or EMA), or in doses currently being assessed during phase 2 clinical trials, and/or according to the maximum allowed dose according to a phase I trial.

The above embodiments, regarding particular amounts, are based on clinically allowed or currently trialed doses of the various compounds described herein, being combined into a pharmaceutical composition as described herein.

For employing the treatments of the present invention, the mutation in genes within a mitochondrial Complex V structural subunit or within an assembly gene of a mitochondrial ATP synthase in a human subject are not only used to identify patients who may need this treatment, but the treatment addresses mechanisms related to disease progression, i.e. by the inhibition or reduction of endogenous cGMP degradation. The spatiotemporal dynamics of cAMP and cGMP pathways depends upon PDE activity, which by breaking phosphodiesteric bonds terminate cyclic nucleotides signaling. PDE5 is an enzyme found, for example, in smooth muscle and neurons that selectively cleaves cGMP and degrades it to 5'-GMP. PDE5 inhibitors are similar in structure to cGMP; they bind competitively to PDE5 and inhibit cGMP hydrolysis, thereby enhancing the effect of NO.

In one embodiment, the PDE5 inhibitor competitively binds to PDE5 and inhibits cGMP hydrolysis in a target cell, such as smooth muscle cells, liver, retina, skeletal muscle, and neurons. Through this inhibiting effect, PDE5 inhibitors reduce or interrupt cGMP degradation leading to increased NO concentrations by each dose administered, wherein NO promotes intracellular calcium excretion from mitochondrial striatal neurons improving the impaired calcium signaling cascade and reducing mitochondrial membrane potential.

In preferred embodiments, said treatment comprises administering avanafil, a derivative or metabolite thereof at 1-500 mg/day to a human subject, preferably at 3-200 mg/day.

In preferred embodiment, avanafil, a derivative or metabolite thereof is administered to a human subject in an amount of 1 mg to 500 mg, preferably 2 mg to 400 mg, more preferably 3 mg to 300 mg, or 4 mg to 200 mg, or 5 mg to 150 mg, preferably about 10 mg, 20 mg, 30 mg, 40 mg, 50 mg, 60 mg, 70 mg, 80 mg, 90 mg, 100 mg, 110mg,120 mg,130 mg, 140 mg or 150 mg or 160 mg or 170 mg or 180 mg or 190 mg or 200 mg per day. In preferred embodiments, the dose of avanafil, a derivative or metabolite thereof is an oral, daily dose of between 1-500 mg, preferably between 50 mg to 200 mg.

In one embodiment, said PDE-5 inhibitor is sildenafil.

In preferred embodiments, said treatment comprises administering sildenafil, a derivative or metabolite thereof at 1-500 mg/day to a human subject, preferably at 3-200 mg/day.

In preferred embodiment, sildenafil, a derivative or metabolite thereof is administered to a human subject in an amount of 1 mg to 500 mg, preferably 2 mg to 400 mg, more preferably 3 mg to 300 mg, or 4 mg to 200 mg, or 5 mg to 150 mg, preferably about 10 mg, 20 mg, 30 mg, 40 mg, 50 mg, 60 mg, 70 mg, 80 mg, 90 mg, 100 mg, 110mg,120 mg,130 mg, 140 mg or 150 mg per day. In preferred embodiments, the dose of sildenafil, a derivative or metabolite thereof is an oral, daily dose of between 1-500 mg, preferably between 25 mg to 100 mg.

In one embodiment, said PDE-5 inhibitor is tadalafil.

In preferred embodiments, said treatment comprises administering tadalafil, a derivative or metabolite thereof at 1-500 mg/day to a human subject, preferably at 3-200 mg/day.

In preferred embodiment, tadalafil, a derivative or metabolite thereof is administered to a human subject in an amount of 1 mg to 500 mg, preferably 2 mg to 400 mg, more preferably 3 mg to 300 mg, or 4 mg to 200 mg, or 5 mg to 150 mg, preferably about 10 mg, 20 mg, 30 mg, 40 mg, 50 mg, 60 mg, 70 mg, 80 mg, 90 mg, 100 mg, 110mg,120 mg,130 mg, 140 mg or 150 mg per day. In preferred embodiments, the dose of tadalafil, a derivative or metabolite thereof is an oral, daily dose of between 1-500 mg, preferably between 5 mg to 20 mg.

In one embodiment, said PDE-5 inhibitor is selected from the group consisting of vardenafil, mirodenafil, udenafil, and lodenafil.

In one embodiment, said PDE5 inhibitor is administered:
a) At a dosage of 0.1-10 mg/kg/day to a human subject, preferably at 0.5-5 mg/kg/day, more preferably at 1-2 mg/kg/day.

In one embodiment, said PDE5 inhibitor is administered:
b) At a frequency of 2 to 4 times per day, preferably 4 times daily, wherein the route of administration is subcutaneously, intravenously or orally, preferably orally via a tablet of 1mg to 100mg per dose, more preferably 2mg to 80mg per dose, even more preferably more preferably 4mg to 40mg per dose.

In one embodiment, the PDE5 inhibitor sildenafil is administered to a patient suffering from MILS or NARP:
a) At a dosage of 1-2 mg/kg/day, and/or
b) At a frequency of 4 times per day, wherein the route of administration is orally via a tablet of 4mg to 40mg per dose.

In one embodiment, the PDE5 inhibitor tadalafil is administered to the patient suffering from MILS or NARP:
a) At a dosage of 1-2 mg/kg/day, and/or
b) At a frequency of 4 times per day, wherein the route of administration is orally via a tablet of 4mg to 40mg per dose.

In one embodiment, the PDE5 inhibitor avanafil is administered to the patient suffering from MILS or NARP:
a) At a dosage of 1-2 mg/kg/day, and/or
b) At a frequency of 4 times per day, wherein the route of administration is orally via a tablet of 4mg to 40mg per dose.

In one embodiment, the PDE5 inhibitor sildenafil is administered to the patient having one or more mutations in the MT-ATP6 or the MT-ATP8 gene:
a) At a dosage of 1-2 mg/kg/day, and/or
b) At a frequency of 4 times per day, wherein the route of administration is orally via a tablet of 4mg to 40mg per dose.

In one embodiment, the PDE5 inhibitor tadalafil is administered to the patient having one or more mutations in the MT-ATP6 or the MT-ATP8 gene:
a) At a dosage of 1-2 mg/kg/day, and/or
b) At a frequency of 4 times per day, wherein the route of administration is orally via a tablet of 4mg to 40mg per dose.

In one embodiment, the PDE5 inhibitor avanafil is administered to the patient having one or more mutations in the MT-ATP6 or the MT-ATP8 gene:
a) At a dosage of 1-2 mg/kg/day, and/or
b) At a frequency of 4 times per day, wherein the route of administration is orally via a tablet of 4mg to 40mg per dose.

In some embodiments, said treatment comprises administering a PDE5 inhibitor, a derivative or metabolite thereof at 1-500 mg/day to a human subject, preferably at 3-200 mg/day.

In some embodiments, said treatment comprises administering sildenafil, a derivative or metabolite thereof at 3-200 mg/day to the patient having one or more mutations in the MT-ATP6 or the MT-ATP8 gene.

In some embodiments, said treatment comprises administering tadalafil, a derivative or metabolite thereof at 3-200 mg/day to the patient having one or more mutations in the MT-ATP6 or the MT-ATP8 gene.

In some embodiments, said treatment comprises administering avanafil, a derivative or metabolite thereof at 3-200 mg/day to the patient having one or more mutations in the MT-ATP6 or the MT-ATP8 gene.

In one embodiment, PDE5 inhibitor, a derivative or metabolite thereof is administered to a human subject in an amount of 1 mg to 500 mg, preferably 2 mg to 400 mg, more preferably 3 mg to 300 mg, or 4 mg to 200 mg, or 5 mg to 150 mg, preferably about 10 mg, 20 mg, 30 mg, 40 mg, 50 mg, 60 mg, 70 mg, 80 mg, 90 mg, 100 mg, 110mg,120 mg,130 mg, 140 mg or 150 mg per day. In preferred embodiments, the dose of PDE5 inhibitor, a derivative or metabolite thereof is an oral, daily dose of between 1-500 mg, preferably between 5 mg to 150 mg.

In some embodiments, the treatment comprises administration of PDE5 inhibitor, a derivative or metabolite thereof 3 times daily for more than one day, more than one week, more than one month, more than one year, preferably 3 times daily lifelong, wherein the route of administration can be a tablet, chewable tablets, meltable tablets or a drinking solution, preferably via a tablet 1 to 100 mg, preferably 1 to 50 mg per dose, via oral delivery or via a gastric tube.

In some embodiments, the PDE5 inhibitor, a derivative or metabolite thereof, the solid or the liquid formulation contain 50 mg active pharmaceutical ingredient per ml solution or per tablet.

In one embodiment, for example, the PDE5 inhibitor and the method of the present invention is described for use treatment and/or prevention of mitochondrial Complex V deficiency related diseases in the human subject by administering the PDE5 inhibitor, or a pharmaceutically acceptable salt, solvate, or composition thereof. In one embodiment, the PDE5 inhibitor may be administered orally.

The PDE5 inhibitor may be administered orally and may be administered together with one or more therapeutic drugs and/or symptomatic measures to mitigate the suffering of the patients. In one embodiment, the PDE5 inhibitor is administered together with one or more therapeutic drugs, i.e. biotin, riboflavin and/or CoQ10. In one embodiment, administration of the PDE5 inhibitor can be carried out before, after or at the same time as symptomatic measures described herein.

The invention also relates to the combined administration of a therapeutically effective amount of the PDE5 inhibitor and one or more therapeutic drugs described herein.

Another major advantage of treatment described herein, is the minimal incidence of side effects, if any.

In one embodiment, said dosage and frequency is configured to prevent and/or to reduce cGMP degradation to decrease intracellular calcium levels in striatal neuron mitochondria and/or to reduce mitochondrial membrane potential below a level at treatment initiation.

In some embodiments, disturbed intracellular calcium levels in striatal neuron mitochondria and increased mitochondrial membrane potential can lead to a manifestation, progression and/or severe course of mitochondrial Complex V deficiency, particularly for MILS and NARP.

It has proven challenging to elucidate the significance of mitochondrial Complex V deficiency in patients since the mitochondrial membrane potential can be normal in patient derived cells, such as blood cells and fibroblasts, and striatal neurons are not accessible from patients. Therefore, so far, no compounds have been found that have a therapeutic effect on intracellular calcium levels in striatal neuron mitochondria and/or mitochondrial membrane potential.

As shown in the Examples, the inventors have surprisingly succeeded in generating induced pluripotent stem cells from fibroblast cells of patients with mutations in the MT-ATP6 gene (MILS patients) and differentiating them into neuronal progenitor cells. The mitochondrial membrane potential is significantly increased in these neuronal precursor cells from MILS patients, caused by the lack of function of the F1F0-ATPase. The inventors found that elevated levels of mitochondrial membrane potential caused disruption of the calcium signalling pathway, of which components were consistently downregulated in patient neurons, leading to decreased intracellular calcium release. Unexpectedly, the treatment of MILS patient neuronal progenitor cells in vitro with the PDE5 inhibitor, such as sildenafil, avanafil or tadalafil, reduces the mitochondrial membrane potential to levels comparable with controls. Measuring normalization of mitochondrial membrane potential in neuronal progenitors of MILS patients, the inventors were able to demonstrate this in neuronal progenitors derived from MILS patients with different MT-ATP6 mutations.

In one embodiment, said treatment with PDE5 inhibitors sildenafil, avanafil or tadalafil reduce the mitochondrial membrane potential in MILS patients to levels comparable with controls, such as neuronal progenitor cells derived from MILS patients.

In one embodiment, said treatment is administered at a dosage and frequency configured to prevent and/or to reduce cGMP degradation to decrease intracellular calcium levels in striatal neuron mitochondria to a level of at least 5%, 10%, 20%, 30%, 40% or at least 50% below a level at treatment initiation.

In one embodiment, the physiological response of competitively binding to PDE5 can be determined by quantifying mitochondrial membrane potential, intracellular calcium level in striated neurons, and/or lactate levels in cells, blood, plasma, serum, cerebral fluid from a mitochondrial Complex V deficient patient, preferably neuronal progenitor cells derived from iPS transformed fibroblasts from MILS subject, treated with PDE5 inhibitor.

In one embodiment, the physiological response to competitively binding to PDE5 can be determined by evaluating clinical parameters of a mitochondrial Complex V deficient patient treated with PDE5 inhibitor. In some embodiments, the evaluation of clinical parameters includes monitoring the progression of medical disease associated with mitochondrial Complex V deficiency, such as MILS, and the clinical status of said patient. In some embodiments, the clinical parameters for monitoring physiological response comprise ECHO cardiography, need for oxygen/invasive ventilation, need for gastric tube, determining walking distance, regaining of abilities (such as independently sitting, moving, speaking), reaching developmental milestones, reversing cardiomyopathy, gaining muscle strength and/or muscle tone.

In one embodiment, mitochondrial membrane potential and intracellular calcium levels are determined as described in the Examples. Other means are known to one skilled in the art.

In one embodiment, said treatment is administered at a dosage and frequency configured to prevent and/or to reduce cGMP degradation to reduce mitochondrial membrane potential to a level of at least 5%, 10%, 20%, 30%, 40% or at least 50% below a level at treatment initiation. Increase mitochondrial oxygen consumption by a level of at least 5%, 10%, 20%, 30%, 40% or at least 50% above the level at treatment initiation.

Increase mitochondrial ATP production by a level of at least 5%, 10%, 20%, 30%, 40% or at least 50% above the level at treatment initiation.

In one embodiment, the preferred administered dosage and frequency of PDE5 inhibitor (e.g. 20 mg per dose and 60 mg per day) has an inhibitory effect on PDE5 and reducing cGMP degradation,
- wherein said dosage reduces level of mitochondrial membrane potential lower as before treatment initiation, preferably a level of mitochondrial membrane potential at least 20% lower as before treatment initiation, and/or
- wherein said dosage reduces level of intracellular calcium levels in striatal neuron mitochondria lower as before treatment initiation, preferably a level of intracellular calcium levels in striatal neuron mitochondria at least 20% lower as before treatment initiation.

A skilled person is capable, based on the experimental and written support provided herein, to carry out the invention, in combination with common knowledge in the art.

By employing the quantitative measures described in the examples, or by using alternative quantitative means for determining reductions in levels of mitochondrial membrane potential and/or intracellular calcium levels (i.e. in striatal neuron mitochondria, neuronal progenitors), the PDE5 inhibitor and their necessary doses can be determined and adjusted in order to obtain beneficial effects due to the inventive application of the composition.

In one embodiment, the effect of said PDE5 inhibitor to said subjects comprises:
- Preventing and/or inhibiting cGMP degradation and reducing mitochondrial membrane potential to levels comparable to healthy subjects, preferably leading to increased energy capacity and a decrease to disappearing of metabolic crises in patients,
- Reducing the number of paralytic events, motor and sensory neuropathy, metabolic crises events, lactic acidosis, cardiomyopathy, seizures, encephalopathy, stroke-like episodes, endocrine abnormalities, anemia, exercise insufficiency, muscle weakness, loss of muscle tone and/or muscular hypotonia, respiratory insufficiency, Retinitis pigmentosa, ataxia, increased susceptibility to infections, developmental delay, intellectual disability, compared to treatment initiation, and/or
- Preventing and/or reducing the risk of said subject for repeated metabolic crises, palliative care or mortality, or resolves the need of the subject to receive palliative care.

Of note is that in vitro data, as evident from some disclosures in the prior art, cannot always reliably predict the outcome of treatment in human patients. No clinical applications for PDE5 inhibitors are disclosed in the prior art for patients suffering from MILS and NARP. From cell culture data alone, a skilled person would not have had a reasonable expectation of success of obtaining the clinical effects as disclosed in the examples below. The relevant patient group is in significant medical need and the present invention has enabled unexpectedly good therapeutic effects, beyond any reasonable expectations based on earlier in vitro data.

In one embodiment, abnormal mitochondrial membrane potential and intracellular calcium levels in striatal neuron mitochondria may be effectively treated by the administration of from about 1 mg to about 500 mg, preferably 2 mg to about 400 mg, more preferably 3 mg to about 100 mg with PDE5 inhibitor per patient per day.

In one embodiment, paralytic events, motor and sensory neuropathy, metabolic crises events, lactic acidosis, cardiomyopathy, seizures, encephalopathy, stroke-like episodes, endocrine abnormalities, anemia, exercise insufficiency, muscle weakness, loss of muscle tone and/or muscular hypotonia, respiratory insufficiency, Retinitis pigmentosa, ataxia, increased susceptibility to infections, developmental delay, intellectual disability, may be effectively treated by the administration of from about 1 mg to about 500 mg, preferably 2 mg to about 400 mg, more preferably 3 mg to about 100 mg with PDE5 inhibitor per patient per day.

In one embodiment, repeated metabolic crises, palliative care or mortality, or resolves the need of the subject to receive palliative care may be effectively treated by the administration of from about 1 mg to about 500 mg, preferably 2 mg to about 400 mg, more preferably 3 mg to about 100 mg with PDE5 inhibitor per patient per day.

The invention further relates to a pharmaceutical composition comprising a PDE5 inhibitor for use as a medicament in the treatment and/or prevention of a medical condition as described herein, wherein said composition is in admixture with a pharmaceutically acceptable carrier and/or formulated in a pharmaceutically buffered solution.

According to embodiments of the present invention, a composition comprising PDE5 inhibitor or salt thereof as well as a drug product comprising said composition have a therapeutically relevant effect on mitochondrial membrane potential, intracellular calcium signalling, mitochondrial energy supply in cells of a subject, muscular hypotonia, hypertrophic cardiomyopathy, psychomotor delay, encephalopathy, peripheral neuropathy, lactic acidosis, brain stem degeneration, hepatomegaly, feeding ability, developmental delay, walking distance, walking speed, demyelinating neuropathy, urinary incontinence, chronic metabolic acidosis, exercise-induced insufficiency, metabolic crisis, cerebral seizures, respiratory failure, lifespan and survival of a subject.

Single preparations, combinational preparations or compositions are known to the skilled person who is able to evaluate compatible carrier materials and formulation forms suitable for both active compounds in the combination. In some embodiments, the quantity of active ingredient that can be combined with the carrier materials to produce a single dosage varies depending on the patient being treated and the particular route of administration.

Further examples of pharmaceutical compositions, pharmaceutical combinations, treatments, subjects, target genes, and targets are described in detail below.

The features of the invention regarding methods of treatment and diagnosis, and descriptions of the composition comprising PDE5 inhibitors, particularly sildenafil and tadalafil, for use in the treatment of various medical conditions, apply to the composition itself, and vice versa.

The present invention includes multiple aspects, features, and embodiments, wherein these multiple aspects, features, and embodiments may be combined and permuted in any desired manner. These and other aspects, features, and embodiments of the present invention will become apparent by reference to the remainder of this application, including the following detailed description. In addition, various references are provided herein that describe specific compositions and/or methods in greater detail; all such references are incorporated herein by reference in their entirety.

### DETAILED DESCRIPTION

The present invention addresses the problem of inadequate pharmacological treatments for genetically caused mitochondrial Complex V deficiency related disorder or a Leigh Syndrome or a Leigh like syndrome. Current treatments are ineffective or can only slightly alleviate some symptoms and often resulting in palliative care being the last option for these patients. The present invention therefore relates to the treatment or prevention of a mitochondrial Complex V deficiency related disorder or a Leigh Syndrome or a Leigh like syndrome in a human subject.

All words and terms used herein shall have the same meaning commonly given to them by the person skilled in the art, unless the context indicates a different meaning. All terms used in the singular shall include the plural of that term and vice versa.

In accordance with the present disclosure, there is provided herein a composition comprising a PDE5 inhibitor, preferably sildenafil or tadalafil, and in the form of a medicament, having an effect on mitochondrial Complex V gene mutation associated medical conditions and in particular mitochondrial Complex V deficiency associated medical conditions, such as Leigh syndrome, Leigh like syndrome or NARP. It has proven difficult in the art to identify treatments of said medical condition that have a therapeutic effect on mitochondrial membrane potential and intracellular calcium levels in striated neurons.

If such an effect is possible, it may help delay or stop disease progression and possibly reverse an associated symptom, risk for symptom or medical condition, such as a cardiomyopathy, repeated metabolic crises, and symptoms, including paralytic events, motor and sensory neuropathy, metabolic crises events, lactic acidosis, cardiomyopathy, seizures, encephalopathy, stroke-like episodes, endocrine abnormalities, anemia, exercise insufficiency, muscle weakness, loss of muscle tone and/or muscular hypotonia, respiratory insufficiency, Retinitis pigmentosa, ataxia, increased susceptibility to infections, developmental delay, and/or intellectual disability and even enabling and prolong said patients survival. Such an effect is found herein for a composition according to the disclosure, which is further described below.

### Mitochondrial respiratory chain and ATP synthesis by Complex V

A basic mechanism responsible for cellular ATP production is mitochondrial respiration. The transfer of electrons by the specific components of the electron transport chain also leads to the transfer of protons from the mitochondrial matrix into the intermembrane space, creating a proton gradient. This proton gradient is then used to boost the production of metabolic energy in the form of adenosine triphosphate (ATP). ATP production by oxidative phosphorylation in functionally respiring mitochondria is associated with the re-oxidation of NADH via the activity of the electron transport system. In cases where the mitochondrial electron transport chain is impaired (as in mitochondrial diseases), there is thought to be a decrease in ATP production and, under certain conditions, a concomitant accumulation of NADH.

This bioenergetic metabolic pathway consists of five enzyme complexes: NADH:CoQ oxidoreductase (complex I, also referred to as NADH dehydrogenase), succinate:CoQ oxidoreductase (complex II), CoQ:cytochrome c oxidoreductase (complex III, also referred to as cytochrome b-c1 complex), cytochrome c oxidase (complex IV, also referred to as COX), and H+-ATPase (Complex V, also referred to as F0F1-ATP synthetase or simply ATP synthase). Both the nuclear and mitochondrial genomes are required for the assembly of oxidative phosphorylation enzyme complexes I, III, IV, and V, whereas complex II is exclusively nuclear-encoded.

As mentioned, the respiratory enzymes, complexes I, III, and IV, pump protons out of the inner mitochondrial matrix, building up proton pressure outside the "dam" (i.e., membrane). Complex V uses the energy of the proton flow into the matrix to synthesize ATP.

Various mitochondrial disorders result from partial dysfunction of mitochondrial oxidative phosphorylation. Respiratory chain disorders include complex I: NADH dehydrogenase deficiency (NADH-CoQ reductase), complex II: succinate dehydrogenase deficiency, complex III: ubiquinone cytochrome c oxidoreductase deficiency, complex IV: cytochrome c oxidase (COX) deficiency, and Complex V: ATP synthase deficiency. See, e.g., Smeitink, J. A., "Mitochondrial disorders: clinical presentation and diagnostic dilemmas," J. Inherit. Metab. Dis. 2003; 26(2-3):199-207.

Certain degenerative diseases are caused by mutations in mitochondrial DNA (mtDNA). The mitochondrial genome is subject to a high mutation rate, primarily because it is in close proximity to the major source of cellular free radicals (the mitochondrial electron transport chain) and because the mitochondrial organelle lacks an efficient DNA repair system. The mitochondrial genome (16,569 bp, SEQ ID No: 1) essentially encodes only genes related to energy production. It contains the structural genes for seven proteins of complex I of the respiratory chain, a single subunit protein of complex III, three subunits of complex IV, and two subunits of ATP synthase (Complex V).

Mitochondrial ATP synthase (Complex V) synthesizes ATP from ADP and inorganic phosphate using the energy provided by the electrochemical proton gradient (proton motive force) at the inner mitochondrial membrane generated by the electron transport complexes of the respiratory chain. It is an F-type ATPase consisting of two structural domains: the F1 sector, a soluble part located in the mitochondrial matrix, and the F0 sector spanning the inner mitochondrial membrane. Protons enter the matrix from the membrane interstitial space through the F0 sector, which transfers the energy generated by the proton motive force to the F1 sector, where ADP is phosphorylated to ATP by a rotational mechanism. The Table below comprises genes encoding for proteins within Complex V.

**Table 1: Genes encoding for proteins within Complex V.**

| **Gene symbol** | **Gene name** | **Alias symbols** | **Location** |
|---|---|---|---|
| ATP5F1A | ATP synthase F1 subunit alpha | ATP5A, hATP1, OMR, ORM | 18q21.1 |
| ATP5F1B | ATP synthase F1 subunit beta | | 12q13.3 |
| ATP5F1C | ATP synthase F1 subunit gamma | | 10p14 |
| ATP5F1D | ATP synthase F1 subunit delta | | 19p13.3 |
| ATP5F1E | ATP synthase F1 subunit epsilon | | 20q13.32 |
| ATP5MC1 | ATP synthase membrane subunit c locus 1 | | 17q21.32 |
| A TP5MC2 | ATP synthase membrane subunit c locus 2 | | 12q13.13 |
| A TP5MC3 | ATP synthase membrane subunit c locus 3 | | 2q31.1 |
| ATP5ME | ATP synthase membrane subunit e | | 4p16.3 |
| ATP5MF | ATP synthase membrane subunit f | ATP5JL | 7q22.1 |
| ATP5MG | ATP synthase membrane subunit g | ATP5JG | 11q23.3 |
| ATP5MJ | ATP synthase membrane subunit j | MP68, MLQ, 6.8PL | 14q32.33 |
| ATP5MK | ATP synthase membrane subunit k | MGC14697, bA792D24.4, DAPIT, AGP | 1oq24.33 |
| MT-ATP6 | mitochondrially encoded ATP synthase membrane subunit 6 | ATP6, ATPase-6, Su6m | mitochondria |
| MT-ATP8 | mitochondrially encoded ATP synthase membrane subunit 8 | ATP8, A6L, URFA6L | mitochondria |
| ATP5PB | ATP synthase peripheral stalk-membrane subunit b | | 1p13.2 |
| ATP5PD | ATP synthase peripheral stalk subunit d | ATPQ, ATP5JD | 17q25.1 |
| ATP5PF | ATP synthase peripheral stalk subunit F6 | CF6 | 21q21.3 |
| ATP5PO | ATP synthase peripheral stalk subunit OSCP | OSCP, ATPO | 21q22.11 |
| ATP51F1 | ATP synthase inhibitory factor subunit 1 | ATPI, IP, ATPIP, MGC1167, MGC8898 | 1p35.3 |

Genes listed in the Table above are part of the mitochondrial Complex V having a structural, transporting and/or enzymatic function within said Complex V. In one embodiment, mitochondrial Complex V deficiency may be caused by an increased enzymatic activity, decreased enzymatic activity, absent enzymatic activity, an increased transporting activity, decreased transporting activity, absent transporting activity, structural changes of the mitochondrial Complex V, absence of mitochondrial Complex V, and/or absence of one or more proteins within mitochondrial Complex V.

In one embodiment, genes carrying a mitochondrial Complex V deficiency causing sequence variant may be selected from ATP5F1A, ATP5F1B, ATP5F1C, ATP5F1D, ATP5F1E, ATP5MC1, ATP5MC2, ATP5MC3, ATP5ME, ATP5MF, ATP5MG, ATP5MJ, ATP5MK, MT-ATP6, MT-ATP8, ATP5PB, ATP5PD, ATP5PF, ATP5PO, and/or ATP5IF1, wherein one or more genes are carrying at least one mutation within said gene. In one embodiment, one or more mutations may be located outside of at least one of said genes that affects an expression regulatory element of said gene.

### Nucleic acid

The term "nucleic acid" as used herein refers to a polymer containing at least two deoxyribonucleotides or ribonucleotides in single- or double-stranded form, and includes DNA, RNA, and hybrids thereof. DNA may be in the form of, for example, antisense molecules, RNA-DNA duplexes, a PCR product, chimeric sequences, derivatives, and combinations of these groups. RNA can be in the form of small interfering RNA (siRNA), dicer substrate dsRNA, small hairpin RNA (shRNA), asymmetric interfering RNA (aiRNA), microRNA (miRNA), mRNA and combinations thereof. DNA and RNA can form DNA-RNA hybrids. As used herein, the terms "polynucleotide" or "nucleic acid molecule" refers to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

"Gene expression" is the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation. Gene expression is regulated by "expression regulatory element" or "regulatory sequence".

An "expression regulatory element" or "regulatory sequence", as used herein, refer to a segment of a nucleic acid molecule that is capable of increasing or decreasing the expression of certain genes in a cell of an organism. Expression regulatory element are known by a skilled person and comprise, for example, promoter, enhancer, silencer, poly-adenylation signal, histone binding sequences, and CpG islands. "Increased expression" of a certain gene refers to a number of protein molecules expressed from said gene being higher in the cell as compared to without elevated gene expression. "Decreased expression" of a certain gene refers to a number of protein molecules expressed from said gene being lower in the cell as compared to without lowered gene expression.

### Sequence Variation:

"Sequence variant" or "gene mutation" "or "mutation", as used herein, refers to a change in the nucleotide sequence of the genome of an organism, a virus, mitochondrial DNA, and/or extrachromosomal DNA. Mutations result from errors during DNA or viral replication, mitosis, or meiosis, or from other types of DNA damage (e.g., pyrimidine dimers caused by ultraviolet radiation), which can then undergo error-prone repair (especially microhomology-mediated end joining), cause an error in other forms of repair, or cause an error during replication (translesion synthesis). Mutations can also result from insertion or deletion of DNA segments due to mobile genetic elements.

Mutations can result in detectable changes in the observable characteristics (phenotype) of an organism. Mutations in genes can also have no effect, alter the product of a gene, or prevent the gene from functioning properly or completely. They can also occur in non-genetic regions. "Pathogenic" sequence variants refer to disease causing gene mutations. A skilled person knows how to find and identify a sequence variant, in particular the pathogenic sequence variant.

### Homoplasmic or heteroplasmic

"Homoplasmic" as used herein, describes a eukaryotic cell having all identical copies of mitochondrial DNA. In particular, homoplasmic MT-ATP6 mutations occur in all cellular copies of the mitochondrial MT-ATP6 gene on the mitochondrial DNA of a subjects. "Heteroplasmic" as used herein, describes a eukaryotic cell with copies of mitochondrial DNA varying in their nucleic acid sequence. As an example, an MT-ATP6 gene mutation, which is heteroplasmic, occurs in a specific proportion of all mitochondrial MT-ATP6 gene copies present on mitochondrial DNA in an individual's cell. Meaning that in the same cell of a subject mitochondrial DNA with MT-ATP6 gene copies that have a mutation are present as well as MT-ATP6 gene copies that do not carry a mutation. The specific proportion of mutated mitochondrial MT-ATP6 gene copies can be 1%, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%.

"MT-ATP6 gene mutation" refers to any MT-ATP6 gene sequence differing to the reference (wt) sequences SEQ ID No: 1 described herein, or known to a skilled person.

### Upstream/ downstream

"Upstream" and "downstream" as used herein, are employed in the context of the 5'→3' direction of a nucleic acid strand. Upstream refers to nucleotides towards the 5' end and downstream towards the 3' end of any given nucleic acid.

### In-frame, out-of-frame

A fusion product is defined as 'in-frame' when an open reading frame remains intact in the 3'-region downstream of the nucleic acid integration site, regardless of the number of inserted or deleted amino acids at the fusion junction point. A shift in the open reading frame of a protein coding gene sequence refers to "out-of-frame".

### Polypeptides

"Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

### Medical Indications

In one aspect of the present invention there is provided a pharmaceutical composition according to the invention as herein described for use in the treatment of a medical condition associated with mitochondrial Complex V deficiency. Such deficiency may be caused by a gene mutation, wherein the medical condition associated with mitochondrial Complex V deficiency is preferably a Leigh syndrome, a Leigh like syndrome, a Maternally Inherited Leigh syndrome (MILS), or a Neuropathy, Ataxia or Retinitis Pigmentosa (NARP) syndrome. The syndromes are defined by their symptoms and/or gene mutation.

In humans, gene mutations in genes of the mitochondrial Complex V can cause mitochondrial Complex V deficiency that can be mild, progressive, or can be lethal.

"Mitochondrial Complex V deficiency" or "ATP synthase deficiency" refers to a lack of mitochondrial Complex V or a loss or reduction of its function compared to normal, wt, healthy levels. Mitochondrial Complex V deficiency associated diseases comprise Leigh syndrome, a Leigh like syndrome, NARP, MILS, Mitochondrial Complex V deficiency Mitochondrial Type 1, and Mitochondrial Complex V deficiency Mitochondrial Type 2.

Complex V is the last of five mitochondrial complexes that perform multistep oxidative phosphorylation for energy production. Disease gene mutations causing mitochondrial Complex V deficiency impair Complex V formation or function. As a result, Complex V activity is reduced and oxidative phosphorylation is impaired. This particularly affects cells, tissues, and organs having a high energy demand, such as skeletal muscles, the brain, or the heart. Genes involved in mitochondrial Complex V deficiency are located on the nuclear DNA, such as TMEM70, and the mitochondrial DNA, such as MT-ATP6. In case of a mutation in mitochondrial DNA, either the entire mitochondrial DNA of a cell contains the mutation (homoplasmy) or only a part of the mitochondrial DNA of a cell contains the alteration (heteroplasmy). The %age of mutated mtDNA regularly leads to differences in disease severity, syndromes, signs and symptoms of the disease, and the risk of exitus lethalis.

In humans, mitochondrial Complex V deficiency can cause a variety of signs and symptoms affecting many organs and systems of the body, especially the nervous system and the heart. The disorder can occur in infancy or early childhood and can also be life-threatening. Affected individuals may have problems with feeding, slow growth, low muscle tone (hypotonia), extreme fatigue (lethargy) and developmental delays. They tend to have elevated levels of lactic acid in the blood (lactic acidosis), which can cause nausea, vomiting, weakness, and rapid breathing. Affected individuals may also experience high levels of ammonia in the blood (hyperammonemia), leading in some cases to impaired brain function (encephalopathy) and damage to other organs.

Hypertrophic cardiomyopathy is also a common feature of mitochondrial Complex V deficiency and is defined as the thickening (hypertrophy) of the heart muscle, potentially leading to heart failure. People with mitochondrial Complex V deficiency may also have a characteristic pattern of facial features, including a high forehead, arched eyebrows, downturned outer corners of the eyes (downturned palpebral fissures), a prominent bridge of the nose, low-set ears, thin lips, and a small chin (micrognathia).

Patients with mitochondrial Complex V deficiency have specific groups of signs and symptoms that are classified as a specific syndrome, such as Leigh syndrome, Leigh like syndrome, Leigh syndrome, the Leigh like syndrome, the Maternally Inherited Leigh syndrome (MILS), or the Neuropathy, an Ataxia or a Retinitis Pigmentosa (NARP) syndrome.

In one embodiment, the mitochondrial Complex V deficiency comprise Leigh syndrome, Leigh like syndrome, MILS, or NARP syndrome. In one embodiment, pharmaceutical composition for use in the treatment of a medical condition associated with mitochondrial Complex V deficiency comprise treatment with a PDE5 inhibitor.

### Guideline on Mitochondrial DNA-Associated Leigh Syndrome and NARP

A guideline is known to be constantly modified and adapted to the current state of medical and scientific knowledge. The guideline for mitochondrial DNA-Associated Leigh Syndrome and NARP covers the entire patient care setting comprising clinical characteristics, diagnostics, current recommendations on therapy management and palliative treatment as well as genetic counselling. (Thorburn DR, Rahman J, Rahman S. Mitochondrial DNA-Associated Leigh Syndrome and NARP. 2003 Oct 30 [Updated 2017 Sep 28]. In: Adam MP, Ardinger HH, Pagon RA, et al., editors. GeneReviews® [Internet]. Seattle (WA): University of Washington, Seattle; 1993-2021. Available from: https://www.ncbi.nlm.nih.gov/books/NBK1173/)

### Leigh Syndrome

Certain forms of Leigh syndrome may be caused by mitochondrial Complex V deficiency. "Leigh syndrome" is defined by characteristic changes in the brain (necrotizing encephalitis), particularly affecting the basal ganglia and brainstem, which are visualized as T2 signal-intense areas on MRI. The disease frequently manifests in the first year of life, usually associated with a viral or bacterial infection. However, later ages of disease onset are also possible. Clinical symptoms of Leigh syndrome comprise muscle weakness, mental and motor developmental impairment, cerebral seizures, circumscribed paralysis, involuntary movements (dystonia, tremor, and chorea), disturbances in nerve function (neuropathy), and thickening of the heart muscle (hypertrophic cardiomyopathy). It is a progressive disease and 50% of affected children die before reaching the age of 3.

The stringent diagnostic criteria according to Rahman et al (1996) for Leigh syndrome are: (i) progressive neurological disease with motor and mental developmental delay, (ii) clinical signs of brainstem or basal ganglia dysfunction, (iii) elevated lactic acid (lactate) concentrations in the blood and/or cerebrospinal fluid, and (iv) at least one of the following abnormalities:
- Characteristic neuroradiological abnormalities (signal abnormalities in basal ganglia, thalamus, brainstem, dentate nucleus, optic nerve) and/or
- Post mortem: Characteristic neuropathological abnormalities (spongiform degeneration) and demyelination in the above designated areas, and/or
- Characteristic radiological or neuropathological abnormalities in a sibling.

### Leigh like syndrome

"Leigh like syndrome" refers to a milder phenotype of Leigh syndrome in which not all of the diagnostic criteria listed for Leigh syndrome are met, or the localization of neuropathologic changes do not correspond exactly to the distribution patterns described for Leigh syndrome. Leigh-like syndrome is also associated with lesions in the basal ganglia, but also in the white matter. Demyelination symptoms or cerebral and cerebellar atrophies are found. In addition to CNS changes, demyelination of peripheral nerves with subsequent neurogenic muscle atrophy may also be found. Other organs are often affected, such as the heart, liver, and kidney.

Leigh-like syndromes are more difficult to classify than the previously described Leigh syndrome because both the predominant symptomatology and the onset of the disease can vary widely from neonatal to adult age. Thus, fulminant lethal courses of the disease with cardiac or liver failure within the first weeks of life to almost inconspicuous clinical symptoms despite impressive magnetic resonance imaging findings and significant respiratory chain defect can be found at all ages.

### Maternally Inherited Leigh syndrome (MILS)

MILS and NARP are caused by specific mutations affecting the mitochondrial ATPase-6 gene. They only differ in the number of mutated mitochondrial DNA copy numbers. Patients having more than 90% mutated mitochondrial DNA in their cells are classified as suffering from MILS syndrome rather than NARP syndrome. MILS often manifests in the first three months to a year of life. However, onset can occur at any time from birth to adulthood. A later onset is often associated with a slower progression of symptoms. In many cases, MILS first appears after a viral infection.

The specific symptoms and severity of MILS vary greatly between patients. In general, this form of Leigh syndrome, also known as infantile necrotizing encephalopathy, is a progressive neurodegenerative disorder. When it occurs in infancy, the first signs may include poor sucking ability and loss of head control. Other early symptoms may include marked loss of appetite, recurrent vomiting, irritability, constant crying and possibly seizures. Delays in reaching developmental milestones may also occur.

Affected individuals may experience decompensation, which in MILS occurs during illness and is typically associated with progressive loss of skills requiring coordination of mental and muscular activity (psychomotor regression).

If the disorder occurs later in childhood, ataxia, difficulty articulating words (dysarthria), loss of previously acquired intellectual skills, and the onset of mental retardation may be the first signs. Progressive neurological deterioration associated with MILS may be characterized by additional symptoms such as generalized muscle weakness, lack of muscle tone (hypotonia), tremors, movement disorders such as chorea (rapid, involuntary, jerky movements), seizures, infantile spasms, and spasticity, a condition characterized by involuntary muscle spasms that result in slow, stiff movements of the legs. Involuntary muscle contractions (dystonia) may also occur, forcing the body into abnormal, sometimes painful movements and positions.

Patients with MILS may also develop a number of breathing abnormalities, including temporary cessation of spontaneous breathing (apnea), shortness of breath (dyspnea), abnormally rapid breathing (hyperventilation), and/or abnormal breathing patterns. Some infants may also have difficulty swallowing (dysphagia). Vision problems include abnormally rapid eye movements (nystagmus), sluggish pupils, squinting (strabismus), paralysis of certain eye muscles (ophthalmoplegia), damage to the nerves of the eye (optic atrophy), and/or visual disturbances that can lead to blindness.

Some pediatric patients with MILS may experience abnormal enlargement of the heart (hypertrophic cardiomyopathy), overgrowth of the fibrous membrane (asymmetric septal hypertrophy), and disease of the nerves outside the central nervous system (peripheral neuropathy) causing progressive weakness of the arms and legs.

MILS can further cause episodes of lactic acidosis, which is characterized by abnormally high concentrations of lactic acid in the blood, brain, and other tissues of the body.

MILS can also cause life-threatening complications, often due to cardiac or respiratory problems. The aforementioned complications develop by age three in some cases, but can also occur later.

### Neuropathy, Ataxia or Retinitis Pigmentosa (NARP) syndrome

Like MILS, NARP syndrome is also caused by mutation in the MT-ATP6 gene, and is characterized by the symptoms neuropathy, ataxia (gait unsteadiness), and retinal degeneration (retinitis pigmentosa). In most cases, the mutation load for the MT-ATP6 mutation in patients with NARP syndrome is lower than that of Leigh syndrome patients. Patients with NARP syndrome have about 80% or less mutated mitochondrial DNA per cell.

Patients with NARP syndrome exhibit a combination of symptoms. The onset of symptoms is often in early childhood. A long stable period is possible, as are frequent episodes of deterioration, usually associated with viral illness. Common symptoms associated with NARP syndrome include ataxia, migraines, seizures, learning difficulties, and/or delays in reaching developmental milestones such as standing up, crawling, or walking (developmental delays). Patients with NARP syndrome also suffer from sensory neuropathy, proximal muscle weakness, and ocular symptoms such as salt-and-pepper retinopathy and progressive degeneration of the retina (retinitis pigmentosa) with night blindness. Other ocular symptoms include rapid, involuntary eye movements (nystagmus) and/or sluggish pupils. Furthermore, NARP patients may develop hearing loss, progressive external ophthalmoplegia, cardiac excitation conduction disorders such as heart block, dementia, and/or mild anxiety disorder.

An abnormal accumulation of lactic acid in the blood (lactic acidosis) rarely occurs in NARP syndrome and is a distinguishing feature from other mitochondrial disorders.

### Mitochondrial Complex V deficiency causing mutations

Mitochondrial Complex V deficiency related diseases are caused by genetic mutations in mitochondrial Complex V and can be identified by genetic screening of patients at risk for disease.

The compounds disclosed herein can be administered to asymptomatic patients with mutations in genes encoding structural subunits or assembly factors of Complex V who are at risk for developing the clinical symptoms of the disease, and the methods of the invention disclosed herein can be used to treat these patients to suppress the onset of adverse symptoms or reduce the severity of symptoms that may occur. The compounds disclosed herein may be administered to symptomatic patients with mutations in Complex V to treat the disease, and the methods of the invention disclosed herein may be used to treat such patients.

The diagnosis of Leigh syndrome, Leigh Like syndrome, MILS or NARP syndrome and other disorders characterized by mitochondrial Complex V deficiency must be performed by a skilled person trained in this field. The skilled person includes physicians, especially those who have experience with this type of syndromes and identification of characteristic symptoms, as well as human geneticists being trained to identify a mutation causing the syndrome and diagnose it through molecular genetic testing. Mutations on nuclear DNA can be performed using any body cell that has a nucleus and chromosomal DNA contained within it, for example, blood cells containing nuclei. The mutations on the mitochondrial DNA associated with said syndromes can be detected in white blood cells (leukocytes), for example, but also other tissue samples such as skin, skeletal muscle, hair follicles or urinary sediment.

The skilled person knows how to identify mitochondrial and nuclear genes and mutations within these genes causing the Complex V deficiency and/or Leigh syndrome. For example, publically available databases can be used, such as ClinVar database, HGMD database, OMIM database, and MitoMap for screening and verifying genes and mutations, particularly pathogenic and disease-causing mutations.

Genes that cause mitochondrial Complex V deficiency and/or Leigh syndrome comprise ATPAF2, TMEM70, ATP5F1E, ATP5F1A, ATP5F1D, ATP5MD, ATP5MC3, ATP5MK, ATP5PO, MT-ATP6, MC5DM1, MT-ATP8, and/or MC5DM2. In the below Table 2, genes with known disease-causing genetic variation and positions of these variation are exemplarily listed.

**Table 2: Genetic variations with chromosomal or mitochondrial DNA position associated with mitochondrial Complex V deficiency.**

| **Gene ID** | **Database entry ID** | **Genetic variation and chromosomal or mitochondrial DNA position** |
|---|---|---|
| ATPAF2 | OMIM *608918, NM_145691 | chr17:17931590A>T \| c.280T>A \| p.W94R |
| MC5DN1 | | chr17:17942230_17942230deIA \| c.98_98deIT c.133+1G>T \| |
| | OMIM #604273 | p.I33Tfs*32 |
| | | chr17:17921948A>G \| c.785T>C \| p. L262P |
| | | chr17:17942194C>T \| c.133+1G>T \| splice site mutation |
| TMEM70 | OMIM *612418, NM_017866.6 | chr8:74888518T>C \| c.2T>C \| p.M1T |
| MC5DN2 | | chr8:74888621_74888622insT \| c.105_106insT \| p. |
| | OMIM #614052 | V36Cfs*52 |
| | | chr8:74888632C>G \| c.116C>G \| p.A39G |
| | | chr8:74888633_74888634insGT \| c.117_118insGT \| |
| | | S40Vfs*11 |
| | | chr8:74891018C>T \| c.238C>T \| p.R80* |
| | | chr8:74893388A>G \| c.317-2A>G \| splice site change |
| | | chr8:74893409T>A \| c.336T>A \| p.Y112* |
| | | chr8:74893414C>T \| c.341C>T \| p.T114M |
| | | chr8:74893446-74893447 \| c.378_379insT \| p.T127Yfs*4 |
| | | chr8:74893567G>A \| c.494G>A \| p.G165D |
| | | chr8:74893528C>T \| c.455C>T \| p. T152M |
| | | chr8:74893573_74893573deIT \| c.500_500deIT \| p. |
| | | V167Afs*34 |
| | | chr8:74893608T>C \| c.535T>C \| p.Y179H |
| | | chr8:74893650_74893651insCA \| c.577_578insCA \| |
| | | p.V194Qfs*8 |
| | | chr8:74893651C>T \| c.578C>T \| p.T193I |
| | | chr8:74893701A>C \| c.628A>C \| p.T210P |
| | | chr8:74893774A>C \| c.701A>C \| p.H234P |
| | | chr8:74893793_74893796delAGAA \| c.720_723delAGAA \| |
| | | stop codon lost |
| ATP5F1E | OMIM *606153, NM 006886 | chr20:57605482A>G \| c.35A>C \| p.Y12S |
| MC5DN3 | | |
| | _ OMIM #614053 | |
| ATP5F1A | OMIM *164360, NM004046 | chr18:43666973C>G \| c.1176+1G \| splice site change |
| MC5DN4 | | Chr18:43667165C>T \| c.985C>T \| p.R329C |
| | _ OMIM #615228 | Chr18:43667188A>G \| c.962A>G \| p.Y321C |
| | | Chr18:43669787T>C \| c.483+2T>C \| splice site change |
| | | chr18:43669562C>T \| c.620G>A \| p. R207H |
| ATP5F1D | OMIM *603150, NM_001687 | chr19:1242558C>T \| c.245C>T \| p.P82L |
| MC5DN5 | | chr19:1244171T>G \| c.371T>G \| p.M124R |
| | OMIM #618120 | chr19:1244117T>G \| c.317T>G\| p. V106G |
| ATP5MD | OMIM *615204, NM_032747 | chr10:105152127C>G \| c.87+1G>C \| splice site change |
| MC5DN6 | | chr10:105156136C>G |
| | OMIM #618683 | |
| ATP5MC3 | OMIM *602736, NM_001002258 | chr2:176043127G>C \| c.318C>G \| p. N106K |
| ATP5MK | OMIM *615204, | chr10:105152125-105152126insT \| c.87+2_87+3insT \| slice |
| | NM_001206427.2 | site change |
| | | chr10:105152127C>G \| c.87+1G>C \| splice site change |
| | | chr10:105152156T>G \| c.59A>C \| p.N20T |
| ATP5PO | OMIM *600828, NM_001697 | chr21:35286751T>C \| c.87+3A>G \| splice site mutation |
| MT-ATP6 | OMIM *516060, NC_012920 | m.8528T>C \| p.M1T |
| MC5DM1 | | m.8551T>C \| p.F9L |
| | OMIM #500015 | m.8558C>T \| p.A11V |
| | | m.8561C>G \| p.P12R |
| | | m.8561C>T \| p.P12L |
| | | m.8573G>A \| p.G16D |
| | | m.8597T>C \| p.I24T |
| | | m.8606C>T \| p.P27L |
| | | m.8608C>T \| p.P28S |
| | | m.8611_8612insC \| frameshift |
| | | m.8612T>C \| p.L29P |
| | | m.8618_8619insT \| frameshift |
| | | m.8639T>C \| p.I38T |
| | | m.8668T>C \| p.W48R |
| | | m.8691A>G \| p.K55K |
| | | m.8719G>A \| p.G65* |
| | | m.8723G>T \| p.R66L |
| | | m.8741T>G \| p.L72R |
| | | m.8779C>T \| p.L85F |
| | | m.8782G>A \| p.G86* |
| | | m.8783G>A \| p.G86E |
| | | m.8794C>T \| p.H90Y |
| | | m.8795A>G \| p.H90R |
| | | m.8821T>G \| p.S99A |
| | | m.8836A>G \| p.M104V |
| | | m.8839G>C \| p.A105P |
| | | m.8843T>C \| p.I106T |
| | | m.8851T>C \| p.W109R |
| | | m.8881T>C \| p.S119P |
| | | m.8890A>G \| p.K122E |
| | | m.8921G>A \| p.G132D |
| | | m.8932C>T \| p.P136S |
| | | m.8936T>A \| p.L137H |
| | | m.8938A>G \| p.I138V |
| | | m.8950G>A \| p.V142I |
| | | m.8951T>C \| p.V142A |
| | | m.8959G>A \| p.E145K |
| | | m.8969G>A \| p.S148N |
| | | m.8989G>C \| p.A155P |
| | | m.8993T>C \| p.L156P |
| | | m.8993T>G \| p.L156R |
| | | m.8993TG>CA \| frameshift |
| | | m.8999T>C \| p.V158A |
| | | m.9010G>A \| p.A162T |
| | | m.9016A>G \| p.I164V |
| | | m.9017T>C \| p.I164T |
| | | m.9025G>A \| p.G167S |
| | | m.9026G>A \| p.G167D |
| | | m.9029A>G \| p.H168R |
| | | m.9032T>C \| p.L169P |
| | | m.9035T>C \| p.L170P |
| | | m.9041A>G \| p.H172R |
| | | m.9049G>A \| p.G175* |
| | | m.9055G>A \| p.A177T |
| | | m.9058A>G \| p.T178A |
| | | m.9071C>T \| p.S182L |
| | | m.9088T>C \| p.S188P |
| | | m.9098T>C \| p.I191T |
| | | m.9101T>C \| p.I192T |
| | | m.9115A>G \| p.I197V |
| | | m.9127_9128del \| frameshift |
| | | m.9133G>A \| p.E203K |
| | | m.9134A>G \| p.E203G |
| | | m.9139G>A \| p.A205T |
| | | m.9152T>C \| p.I209T |
| | | m.9155A>G \| p.Q210R |
| | | m.9155A>T \| p.Q210L |
| | | m.9166T>C \| p.F214L |
| | | m.9176T>C \| p.L217P |
| | | m.9176T>G \| p.L217R |
| | | m.9185T>C \| p.L220P |
| | | m.9191T>C \| p.L222P |
| | | m.9205_9206del \| p.Ter-M |
| MT-ATP8 | OMIM *516070, NC_012920 | m.8381A>G \| p.T6A |
| MC5DM2 | | m.8393C>T \| p.P10S |
| | OMIM #500015 | m.8403T>C \| p.I13T |
| | | m.8411A>G \| p.M16V |
| | | m.8412T>C \| p.M16T |
| | | m.8414C>T \| p.L17F |
| | | m.8418T>C \| p.L18P |
| | | m.8481C>T \| p.P39L |
| | | m.8490T>C \| p.M42T |
| | | m.8519G>A \| p.E52K |
| | | m.8528T>C \| p.W55R |
| | | m.8529G>A \| p.W55* |
| | | m.8558C>T \| p.P65S |
| | | m.8561C>G \| p.P66A |
| | | m.8561C>T \| p.P66S |

Table 2 shows a selection of mutations as known at the time of disclosure. Further mutations in the listed genes are possible and patients with these mutations can be treated with a PDE5 inhibitor as disclosed herein if the mutation leads to Leigh syndrome, Leigh like Syndrome, MILS or NARP.

Treatment with non-PDE5 inhibitor drugs and/or symptomatic measures depends on symptoms and the syndrome caused by the mutation. Non-PDE5 inhibitor drugs administered in the art to Leigh syndrome patients comprise biotin, riboflavin, CoQ10, dichloroacetate, mitoQ, decanoic acid, resveratrol, nicotinamide, alpha-ketoglutarate, and/or aspartate.

Standard therapy in the art include biotin administration, ketogenic diet, treatment of seizures and anti-seizure prophylaxis, treatment of dystonia with benzhexol, baclofen, gabapentin and other similar drugs such as botulinum toxin, supportive care for congestive heart failure in cardiomyopathy, advice on an appropriate nutritious diet and necessary feeding techniques to achieve adequate daily intake, acidosis that may be treated acutely with sodium bicarbonate or sodium citrate, and/or visual disability assistance. Patient at palliative care may also be treated with lifesaving measures including antiepileptic drugs, barbiturates, anaesthetics, full condition monitoring, and/or emergency treatment during dyspnea or respiratory failure.

In particular, the treatment described herein relates to either reducing or preventing a progressive course or symptoms thereof of the mitochondrial Complex V related diseases described herein by inhibiting cGMP degradation within the cytosol of patients cells, in particular encephalopathy, cerebral seizures, severe leg-related motor-sensory neuropathy, demyelinating neuropathy, mechanical ventilation due to respiratory failure, cardiomyopathy, metabolic derailments, loss of ambulation, sitting, and autonomic care abilities, muscle hypotonia, palliative care, muscle weakness, chronic metabolic acidosis, exercise-induced failure.

The prophylactic therapy as described herein is intended to encompass prevention or reduction of risk of developing or progression of disease course and symptoms thereof in mitochondrial Complex V deficiency related diseases described herein, preferably due to decrease in mitochondrial membrane potential, increase in intracellular calcium release and increase in cellular energy demand in patients cells to levels comparable to normal controls described herein via inhibiting cGMP degradation by PDE5.

The interventional therapy as described herein is intended to encompass prevention or reduction of developing or progressing disease course and symptoms thereof in mitochondrial Complex V deficiency related diseases and death, preferably due to decrease in mitochondrial membrane potential, increase in intracellular calcium release, and increase in cellular energy demand in patients cells to levels comparable to normal controls described herein via inhibiting cGMP degradation by PDE5, such as palliative care, encephalopathy, cerebral seizures, severe leg-related motor-sensory neuropathy, demyelinating neuropathy, mechanical ventilation due to respiratory failure, cardiomyopathy, metabolic derailments, loss of ambulation, sitting, and autonomic care abilities, muscle hypotonia, muscle weakness, chronic metabolic acidosis, exercise-induced failure.

In one embodiment, the treatment described herein also relates to reversing an existing disease progression or existing symptoms thereof of the mitochondrial Complex V deficiency related diseases, preferably by decrease in mitochondrial membrane potential, increase in intracellular calcium release, and increase in cellular energy demand in patients cells to levels comparable to normal controls described via inhibiting cGMP degradation by PDE5, such as palliative care, encephalopathy, cerebral seizures, severe leg-related motor-sensory neuropathy, demyelinating neuropathy, mechanical ventilation due to respiratory failure, cardiomyopathy, metabolic derailments, loss of ambulation, sitting, and autonomic care abilities, muscle hypotonia, muscle weakness, chronic metabolic acidosis, exercise-induced failure.

As used herein, a "patient with symptoms of a mitochondrial Complex V deficiency" is a subject who presents with one or more of, without limitation, characteristic changes in the brain necrotizing encephalitis, muscle weakness, ataxia, mental and/or motor developmental impairment, psychomotor regression, mental and/or motor developmental delay, cerebral seizures, circumscribed paralysis, involuntary movements (dystonia, tremor, chorea), lesions in the basal ganglia, lesions in the white matter, disturbances in nerve function (neuropathy), peripheral neuropathy, progressive weakness of the arms and legs, hypertrophic cardiomyopathy, asymmetric septal hypertrophy progressive neurological disease with motor and mental developmental delay, clinical signs of brainstem or basal ganglia dysfunction, demyelination symptoms, cerebral atrophies, cerebellar atrophies, neurogenic muscle atrophy, elevated lactic acid (lactate) concentrations in the blood, brain and/or cerebrospinal fluid, neuroradiological abnormalities (signal abnormalities in basal ganglia, thalamus, brainstem, dentate nucleus, and/or optic nerve).

Further symptoms may be difficulty articulating words (dysarthria), loss of previously acquired intellectual skills, poor sucking ability, loss of head control, marked loss of appetite, recurrent vomiting, irritability, constant crying, possibly seizures, breathing abnormalities, including temporary cessation of spontaneous breathing (apnea), shortness of breath (dyspnea), abnormally rapid breathing (hyperventilation), abnormal breathing patterns, difficulty swallowing (dysphagia, abnormally rapid eye movements (nystagmus), sluggish pupils, squinting (strabismus), paralysis of certain eye muscles (ophthalmoplegia), damage to the nerves of the eye (optic atrophy), and/or visual disturbances including blindness. Additional symptoms such as generalized muscle weakness, lack of muscle tone (hypotonia), tremors, movement disorders such as chorea (rapid, involuntary, jerky movements), seizures, infantile spasms, and spasticity, a condition characterized by involuntary muscle spasms that result in slow, or stiff movements of the legs, dystonia and/or painful movements may also occur. Other characteristics and symptoms may be disease worsening and/or progression through or after a viral or bacterial infection. Life-threatening complications, often due to cardiac or respiratory problems, occur frequently in patients with symptoms of mitochondrial Complex V deficiency.

As used herein, a "patient with symptoms of a Leigh syndrome" is a subject who presents with one or more of, without limitation, necrotizing encephalitis (particularly affecting the basal ganglia and brainstem), disease manifestation and progression associated with a viral or bacterial infection, muscle weakness, mental and motor developmental impairment, cerebral seizures, circumscribed paralysis, involuntary movements (dystonia, tremor, and chorea), disturbances in nerve function (neuropathy), and thickening of the heart muscle (hypertrophic cardiomyopathy, progressive neurological disease with motor and mental developmental delay, clinical signs of brainstem or basal ganglia dysfunction, elevated lactic acid (lactate) concentrations in the blood and/or cerebrospinal fluid, neuroradiological abnormalities (signal abnormalities in basal ganglia, thalamus. Brainstem, dentate nucleus, optic nerve), neuropathological abnormalities (spongiform degeneration) and/or demyelination (in basal ganglia, thalamus, brainstem, dentate nucleus, and/or optic nerve).

As used herein, a "patient with symptoms of a Leigh like syndrome" is a subject who presents with one or more of, without limitation, a milder phenotype of Leigh syndrome in which not all of the diagnostic criteria listed for Leigh syndrome are met, lesions in the basal ganglia, lesions in the white matter, demyelination, cerebral atrophies, cerebellar atrophies, demyelination of peripheral nerves, neurogenic muscle atrophy, The course of the disease may be lethal due to cardiac or liver failure.

As used herein, a "patient with symptoms of a Maternally Inherited Leigh syndrome" is a subject who presents with one or more of, without limitation, manifestation and disease progression associated with a viral infection, necrotizing encephalopathy, demyelinating neuropathy, ataxia, dysarthria, urinary incontinence, chronic metabolic acidosis, exercise-induced insufficiency, muscle weakness, delays in reaching developmental milestones, psychomotor regression, mental retardation, lack of muscle tone (hypotonia), tremors, movement disorders such as chorea (rapid, involuntary, jerky movements), seizures, infantile spasms, spasticity, slow and stiff movements of the legs, asymmetric septal hypertrophy, peripheral neuropathy, progressive weakness of the arms and legs, dystonia and/or painful movements due to dystonia. Other symptoms may be poor sucking ability loss of head control, loss of appetite, recurrent vomiting, irritability, constant crying, and/or episodes of lactic acidosis. Life-threatening complications and death, often due to cardiac or respiratory problems, may also occur.

As used herein, a "patient with symptoms of a Neuropathy, Ataxia or Retinitis Pigmentosa (NARP) syndrome" is a subject who presents with one or more of, without limitation, neuropathy, ataxia (gait unsteadiness), and retinal degeneration (retinitis pigmentosa), manifestation and disease progression associated with viral infections, long non-progressive disease period, deterioration episodes, deterioration episodes associated with viral infection, ataxia, migraines, seizures, learning difficulties, and/or developmental delays. Further symptoms may be sensory neuropathy, proximal muscle weakness, and ocular symptoms such as salt-and-pepper retinopathy and progressive degeneration of the retina (retinitis pigmentosa) with night blindness, nystagmus, sluggish pupils, hearing loss, progressive external ophthalmo-plegia, cardiac excitation conduction disorders such as heart block, dementia, and/or mild anxiety disorder. The symptom lactic acidosis rarely occurs in NARP syndrome.

In some embodiments, symptoms of Leigh syndrome are psychomotor retardation, seizures, muscle hypotonia, muscle weakness, ataxia, eye abnormalities including vision loss, difficulty in swallowing, lactic acidosis, lesions of the basal ganglia, thalamus, brain stem, and spinal cord, degeneration of the basal ganglia, thalamus, brain stem, and spinal cord, and/or life-threatening cardiac complications.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

### Subject

As used herein, the "patient" or "subject" may be a vertebrate. In the context of the present invention, the term "subject" includes both humans and animals, particularly mammals, and other organisms. The term "patient" refers to animals, preferably mammals, especially humans and includes adults and children, males and females. Children include neonates, infants, and adolescents. The term "patient" also refers to a "subject" suffering from or suspected of suffering from Leigh syndrome or Leigh like syndrome or MILS or NARP or mitochondrial Complex V deficiency.

As used herein, the term "subject" refers to a mammal, such as humans, but can also be another animal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like). The term "patient" refers to a "subject" suffering from or suspected of suffering from the Leigh syndrome, Leigh-like syndrome, NARP, MILS, or the medical condition associated with mitochondrial Complex V deficiency.

In one embodiment, treatment criteria comprise a positive laboratory-confirmed mutation within a mitochondrial Complex V gene and/or a patient being diagnosed with Leigh Syndrome before treatment. In one embodiment, the onset of Leigh syndrome, Leigh-like syndrome, NARP, MILS, or the medical condition associated with mitochondrial Complex V deficiency may be at birth, <1, <2, <3, <4, <5, <6, <7, <8, <9, <10, <11, <12, <13, <14, <15, <16, <17, <18,<19, <20 years or above, preferably <5 years.

### Inhibitors

According to the present invention, an "inhibitor" in the context of "PDE5 inhibitor" is considered to be any agent, substance, compound, molecule, or other means that results in slowing, repressing, blocking, or otherwise interfering with or negatively affecting the activity, function, expression, or signaling that said target induces, performs, or exhibits in the absence of the inhibitor.

The terms "agent", "substance", "compound", "molecule" can be used interchangeably. For example, phosphodiesterase 5 enzyme inhibitors, i.e. sildenafil and tadalafil, may effect activity of a target enzyme involved in cleaving the phosphoric ester bond into cGMP for 5'-GMP production within a cell, either directly or indirectly. The inhibitors as described herein may also be termed "agents". References to the "agents" in the context of the combinations and methods described herein are to be understood as PDE5 enzymatic activity and cGMP degradation inhibitor. Preferred inhibitors are those described herein.

### PDE and PDE5 Inhibitors

Phosphodiesterases (PDEs) are intracellular enzymes that specifically catalyze the hydrolysis of the second messengers cAMP and cGMP to the inactive metabolites AMP and GMP. PDEs can be divided into Class I and Class II, which have no apparent sequence similarity. Class I includes all known mammalian PDEs and consists of at least 10 families that have arisen from separate genes. Most families contain more than one gene and most genes encode more than one messenger RNA (mRNA) by alternative splicing or alternative transcription start sites. PDE4, PDE7, and PDE8 are highly specific for hydrolysis of cAMP, while PDE5, PDE6, and PDE9 are highly specific for cGMP. PDE1, PDE2, PDE3, and PDE10 have mixed specificity.

Each PDE has a conserved catalytic domain of approximately 270 amino acids with a high degree of conservation (25-30%) of amino acid sequence between PDE families that is carboxyl-terminal to its regulatory domain. Cyclic nucleotides are degraded by PDE-catalyzed hydrolytic cleavage of the 3'-phosphodiester bond, resulting in the formation of the corresponding inactive 5'-monophosphate.

Only PDE5 exclusively catalyses the breakdown of cGMP. By balancing cGMP production through guanylate cyclases, PDE5 is able to lower cGMP levels very effectively. As a result, PDE5 inhibition increases intracellular cGMP levels and initiates a cGMP-driven response cascade. Three different isoforms of PDE5A are known, PDE5A1, PDE5A2 and PDE5A3. All PDE5 variants differ only at the Λ/-terminal end. PDE5A1 appears to be the predominant form expressed in most PDE5-containing tissues. PDE5A2 contains a much shorter Λ/-terminal amino acid fragment and has also been detected in several animal species. PDE5A3 has only been detected in human tissues, based on RT-PCR data. See Rybalkin et al, Circ Res, vol 93, pp 280-291 (2003). PDE5 is highly specific for cGMP hydrolysis and contains two homologous Λ/-terminal regulatory domains, recently defined as GAF A and GAF B. See Martinez et al, Proc Natl Acad Sci USA, vol 99, pp 13260-13265 (2002). PDE5 is activated directly by the binding of cGMP to its GAF A domain. Without cGMP binding, PDE5 is in a non-activated state. Only activated PDE5 is phosphorylated by cGMP-dependent protein kinase (PKG) (Ser-92).

"PDE 5 inhibitors" refers to cyclic guanosine-3',5'-monophosphate type 5 cGMP inhibitors (or phosphodiesterase 5 (PDE5) inhibitors), sometimes referred to herein as PDE V or PDE5 inhibitors. Suitable PDE5 inhibitors for use according to the present invention include sildenafil, tadalafil, and vardenafil. These PDE5 inhibitors are currently approved for the treatment of erectile dysfunction. PDE5 inhibitors increase cyclic guanosine monophosphate (cGMP) levels, which has a neuroprotective effect via activation of protein kinase 1 (PKG1) and improves long-term potentiation.

| **Substance** | **CAS Nummer** | **Structural formula** |
|---|---|---|
| **Sildenafil** | 139755-83-2 | 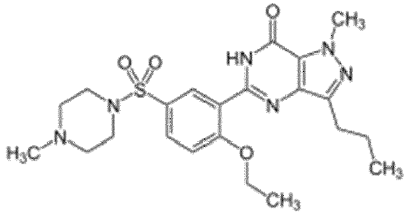 |
| **Sildenafil citrate** | 171599-83-0 | 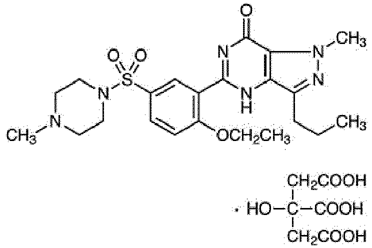 |
| **Tadalafil** | 171596-29-5 | 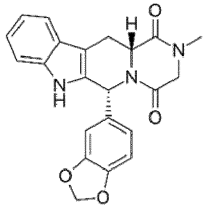 |
| **Vardenafil** | 224785-90-4 | 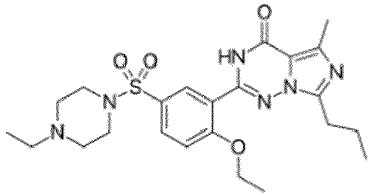 |
| **Mirodenafil** | 862189-95-5 | 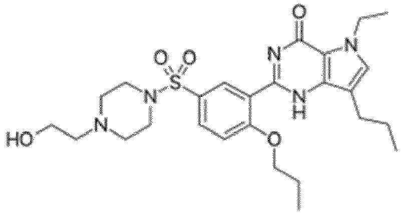 |
| **Udenafil** | 268203-93-6 | 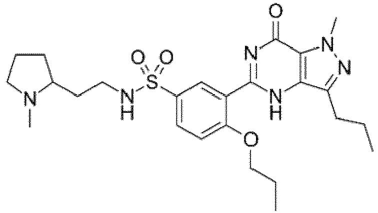 |
| **Lodenafil** | 398507-55-6 | 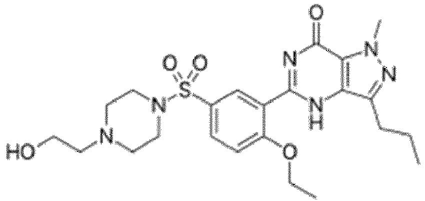 |
| **Xanthoanthrafil** | 247568-68-9 | 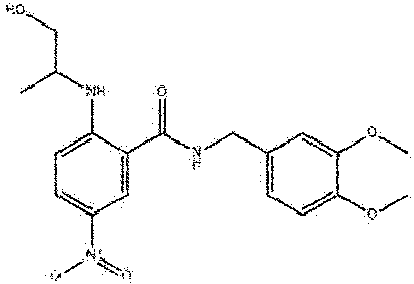 |
| **Homosildenafil** | 642928-07-2 | 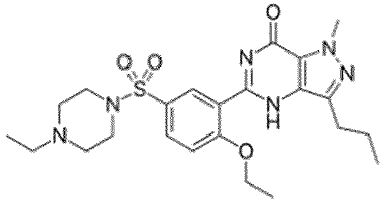 |
| **Hydroxyacetildenafil** | 147676-56-0 | 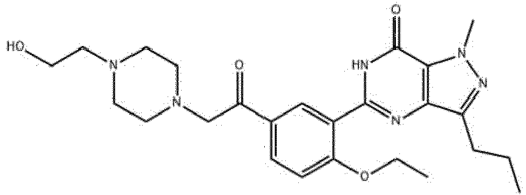 |
| **Aildenafil** | 496835-35-9 | 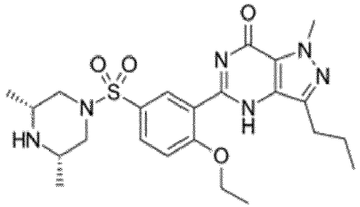 |
| **Zaprinast** | 37762-06-4 | 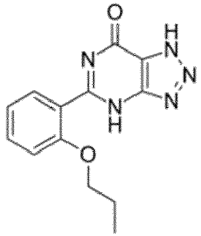 |
| **Icariin** | 489-32-7 | 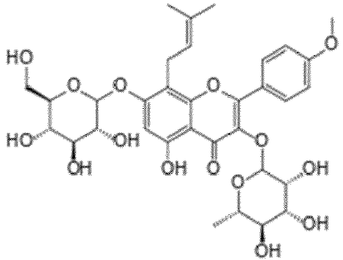 |

PDE5 inhibitors increase intracellular cGMP concentrations by decreasing degradation of the molecule and thus lead to vasodilatation. cGMP leads to increased NO concentrations, NO-itself promotes intracellular calcium excretion from mitochondrial striatal neurons and thus may at least partially improve the impaired calcium signaling cascade. (Horn TFW, Wolf G, Duffy S, Weiss S, Keilhoff G, MacVICAR BA. Nitric oxide promotes intracellular calcium re-lease from mitochondria in striatal neurons. FASEB J 2002;16:1611-22.) Avanafil belongs to the larger group of substances known as phosphodiesterase 5 (PDE5) inhibitors. Avanafil has so far only been used in male erectile dysfunction and no dose data and experience exist for long-term use.

In some embodiments, the treatment comprises treatment of Leigh syndrome, Leigh-like syndrome, and NARP syndrome and disorders caused by or associated with a mutation in a functional subunit of mitochondrial Complex V (F1F0-ATPase) with a PDE5 inhibitor.

Sildenafil citrate and its metabolites are the first selective PDE5 inhibitor. It is a potent inhibitor of PDE5 (IC50 of 3.9 nM), with a high selectivity (>1000-fold) for human PDE5 over humanPDE2, PDE3, and PDE4 and moderate selectivity (>80-fold) over PDE1. Sildenafil, however, is only approximately 10-fold as potent for PDE5 as for PDE6, which is found in the photoreceptors of the human retina. This lower selectivity toward PDE6 is presumed to be the cause for color vision abnormalities observed with high doses or plasma levels of sildenafil. Both sildenafil and its major active metabolite (N-desmethyl sildenafil) are highly bound to plasma proteins (≈96%), and the protein binding is independent of drug concentrations. Plasma concentrations of sildenafil and its N-desmethyl metabolite were re-ported to decline bi-exponentially, with a mean terminal half-life of 3 to 5 hours for both of them, independent of the route of administration.

The substance sildenafil (Pfizer, Revatio^{®}) has been approved for the German market since October 28, 2005; among other things, for the treatment of erectile dysfunction in men. The approval relates to the treatment of adult patients with pulmonary arterial hypertension (PAH) in WHO functional classes II and III for the improvement of exercise capacity. The dosage forms are additionally approved for the treatment of pediatric patients aged 1 to 17 years with pulmonary arterial hypertension. Several studies exist on the use of sildenafil for the treatment of persistent pulmonary hypertension of the premature or newborn infant. There is no approval for this indication in Germany. Other studies with positive outcome of treatment of children with sildenafil are bronchiopulmonary dysplasia, congenital diaphragmatic hernia, Eisenmenger complex, and lymphatic malformations.

Tadalafil is currently approved only for the treatment of erectile dysfunction and for the treatment of benign prostatic syndrome in adult men. Tadalafil is a selective and potent inhibitor of PDE5 with an IC50 of 0.94 nm. It exhibits high selectivity toward PDE5 compared to other PDEs: >700-fold relative to PDE6, >10 000-fold relative to PDE1-4 and 7-10, and >5-fold relative to PDE11. Tadalafil is structurally different from both sildenafil and vardenafil, and the different structures are reflected in distinct differences in the clinical pharmacology profiles of these drugs. Like sildenafil, tadalafil was developed initially for use in cardiovascular disease. Metabolites of tadalafil, such as methylcatechol and methylcatechol glucuronide me-tabolites are clinically inactive at observed metabolite concentrations.

Vardenafil hydrochloride was the first second-generation PDE5 inhibitor. Vardenafil has a high potency in vitro (IC50= 0.1 nm-0.7 nm) and a high selectivity for the inhibition of PDE5 compared with the otherknown phosphodiesterases (>15-fold relative to PDE6,>130-fold relative to PDE-1, >300-fold relative toPDE11, and >1000-fold relative to PDE2, 3, 4, 7, 8, 9, and 10). Unlike sildenafil and tadalafil, vardenafil was developed from the outset specifically for use as an erectogenic agent. Oral doses of 5, 10, and 20 mg vardenafil given no morethan once daily have been efficacious in clinical trials. Vardenafil is extensively metabolized, with more than14 metabolites identified. The major metabolite, M1 ,and 2 minor metabolites, M4 and M5, as well as their respective glucuronides, are all a result of the degradation of vardenafil's piperazine ringThe major circulating metabolite, M1,has 28% of vardenafil's potency for PDE5 inhibition,while M4 and M5 possess 5.6% and 4.9%, respec-tively.

### Treatment and therapeutic methods

As used herein "treatment" or "treating," includes any beneficial or desirable effect on the symptoms or pathology of a disease or pathological condition, and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In some embodiments, a time interval between treatment or prevention comprises a period of several hours (2, 3, 4, 5, 6, 7, 8, 9, 10, 11 or 12) several days (2, 3, 4, 5, 6, or 7), several weeks (1, 2, 3, 4, 5, 6, 7, or 8), or months (2, 3, 4, 5, or 6), preferably 4 weeks to 3 months. In some embodiments, the time interval from one treatment or prevention to the next subsequent treatment can be the same, nearly the same or can change.

In some embodiments, the treatment comprises administration of PDE5 inhibitor, a derivative or metabolite thereof, one or two or three or four times daily for more than one day, more than one week, more than one month, more than one year, preferably 3 times daily, lifelong.

In one embodiment, a method of treating a Leigh syndrome or mitochondrial Complex V deficiency related condition in a subject in need thereof comprises administering an effective amount, e.g., therapeutically effective amount of a composition comprising the PDE5 inhibitor contemplated herein. The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation or transplantation. In a preferred embodiment, compositions are administered orally in tablet form or drinking solution.

### Host cell and target cell

The term "host cell" and "target cell", as used herein, refers to a single cell or cell culture that may be or has been a recipient of at least one of the agents described herein, individually or in combination. Host cells include progeny of a single host cell, which progeny may not necessarily be completely identical (in morphology or overall DNA complement) to the original parent cell due to natural, random or intentional mutations and/or changes. In one embodiment, the host cell also refers to a cell into which an infectious agent (e.g. a virus) has invaded or is capable of invading.

### Pharmaceutical Compositions and Methods of administration

The present invention also relates to a pharmaceutical composition comprising the compounds described herein. The invention also relates to pharmaceutically acceptable salts of the compounds described herein, in addition to enantiomers and/or tautomers of the compounds described.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated or they may be coated by known techniques to delay disintegration and absorption in the gastrointestinal tract and thereby provide a sustained action over a longer period.

In one embodiment, the pharmaceutical composition comprising the PDE5 inhibitor, preferably sildenafil, described herein is supplied to the subject three times daily as tablet or liquid formulation, preferably 1-2 mg/kg/day sildenafil, preferably administered either as tablet or drinking solution orally or via a gastric tube.

Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally, topically, transdermally, or rectally, sublingually, intramuscular, subcutaneously, or intravenously in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of the invention as the/an active agent, and, in addition, may include other medicinal agents, pharmaceutical agents, carriers, adjuvants, etc.

Dosage levels of the order of from about 0.1 mg to about 10 mg per kilogram of body weight per day are useful in the treatment of the indicated conditions. For example, MILS and NARP may be effectively treated by the administration of from about 1 mg to about 500mg, preferably 2 mg to about 400 mg, more preferably 3 mg to about 100 mg, even more preferably 12 mg to about 120 mg, of the PDE5 inhibitor, preferably sildenafil, per patient per day.

Dosage levels of the order of from about 0.1 mg to about 10 mg per kilogram of body weight to a human subject, preferably at 0.5-5 mg/kg/day, more preferably at 1-2 mg/kg/day, are useful in the treatment of the indicated conditions.

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 500 mg of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies.

The invention relates also to a process or a method for the treatment of the mentioned pathological conditions. The compounds of the present invention can be administered prophylactically or therapeutically, preferably in an amount that is effective against the mentioned disorders, to a warm-blooded animal, for example a human, requiring such treatment, the compounds preferably being used in the form of pharmaceutical compositions.

In some embodiments, a patient may receive therapy for the treatment and/or management of the medical condition associated with mitochondrial Complex V deficiency before, during or after the administration of the therapeutically effective regimen of the compound of the invention, or a pharmaceutically acceptable salt thereof. Non-limiting examples of such a therapy include biotin, CoQ10, pain management, anti-inflammatory drugs, oxygen supply, immunotherapy, targeted therapy (i.e. therapy directed toward a specific target or pathway), and any combination thereof. In some embodiments, the patient has not previously received a therapy for the treatment and/or management of mitochondrial Complex V deficiency.

### Therapeutically effective and therapeutically relevant

The phrase "therapeutically effective" is intended to include, within the scope of sound medical judgment, excessive toxicity, irritation, allergic reactions, and/or other problems or complications, but commensurate with a reasonable benefit/risk ratio. As used herein to refer to the compounds, compositions, combinations and/or dosage forms suitable for use in contact with a subject that produces a result that in and of itself helps to treat and/or cure a disease.

A "therapeutically relevant amount", "therapeutically relevant dosage" or "therapeutically effective amount" of an agent or therapeutic means, such as a PDE5 inhibitor, i.e. sildenafil and tadalafil, is an amount sufficient to produce the desired effect, e.g., inhibition of cGMP degradation relative to the cGMP levels detected in the absence of a PDE5 inhibitor. Inhibition of cGMP degradation is achieved when the level obtained with an PDE5 inhibitor relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring cGMP degradation include, for example, assaying cGMP using techniques known to those skilled in the art, such as ELISA, enzyme function, mass spectrometry, LC/GC, and phenotypic assays known to those skilled in the art.

By "decrease," "decreasing," "reducing," "lower" or "diminishing" of cGMP degradation by a PDE5 inhibitor, i.e. sildenafil and/or tadalafil, is meant as a detectable rise in intracellular cGMP concentration to a given PDE5 inhibitor, i.e. sildenafil and/or tadalafil. The extent of decrease in intracellular cGMP degradation by a PDE5 inhibitor, i.e. sildenafil and/or tadalafil, may be determined relative to the intracellular cGMP level without the presence of a PDE5 inhibitor, i.e. sildenafil and/or tadalafil, preferably before initiation of treatment with a PDE5 inhibitor, i.e. sildenafil and/or tadalafil. A detectable increase intracellular cGMP level may be about 5%, 10%, 15%, 20%, 25%, 30%, 35%, 40%, 45%, 50%, 55%, 60%, 65%, 70%, 75%, 80%, 85%, 90%, 95%, or 100% than the intracellular cGMP level detected before initiation of treatment with PDE5 inhibitor, i.e. sildenafil and/or tadalafil. A decrease in mitochondrial membrane potential and increase of cellular cGMP levels by the PDE5 inhibitor is typically understood and measured by a decrease in cGMP degradation by a responder cell in vitro or in vivo.

"Systemic administration," as used herein, refers to the administration of a composition that results in broad biodistribution of PDE5 inhibitor, preferably sildenafil, within an organism. Systemic administration means exposing a therapeutic amount of an agent to preferred parts of the body. Systemic administration of the composition may be accomplished by any means known in the art, e.g., intravenously, subcutaneously, intraperitoneally.

### Combined administration:

According to the present invention, the term "combined administration", otherwise known as coadministration or joint treatment, encompasses in some embodiments the administration of separate formulations of the compounds described herein, whereby treatment may occur together, within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g. orally by ingesting separate tablets simultaneously. A combination medicament, such as a single formulation comprising multiple agents disclosed herein, and optionally additional anti-viral and/or anti-inflammatory medicaments, may also be used in order to co-administer the various components in a single administration or dosage.

A combined therapy or combined administration of one agent may occur together or precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-24 h of each other and, more preferably, within about 3-12 h of each other, with a delay time of only about 6 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

### SEQUENCES

### Preferred mitochondrial nucleic acid sequences of the invention:

| **Seq_ID NO** | **Database entry ID** | **Homo sapiens mitochondrion, complete genome** |
|---|---|---|
| 1 | NCBI Reference Sequence: | >NC_012920.1 Homo sapiens mitochondrion, complete genomeG |
| | NC_012920.1 | |

### FIGURES

The invention is further described by the figures. These are not intended to limit the scope of the invention.

Short description of the figures:
**Figure 1****: Compound high-content screening (HCS) Assessment of cell viability in NPCs_ATP6.**
**Figure 2****: Mitochondrial membrane potentials (MMPs) in NPCs from MILS patients.**
**Figure 3****: Mitochondrial membrane potentials (MMPs) in NPCs from MILS patients and Leigh syndrome patient with NDUFS4 mutation.**

### Detailed description of the figures:

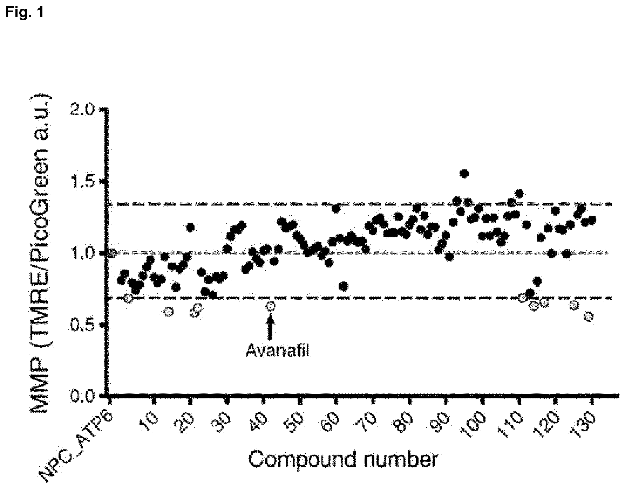

**Figure 1****: Compound high-content screening (HCS) Assessment of cell viability in NPCs_ATP6.** Screening of 130 compounds (all 1 mM in 0.04% DMSO overnight). Effect of different compounds at 1 mM concentration on mitochondrial membrane potential (MMP) of patient neural progenitor cells (NPCs) NPC_ATP6. NPC_ATP6 treated overnight with 0.04% DMSO (dark orange) was used as baseline. The dashed line indicates the range of ±2SD distance from the baseline. Shown here are the average values (two biological replicates, each with two technical duplicates per compound).

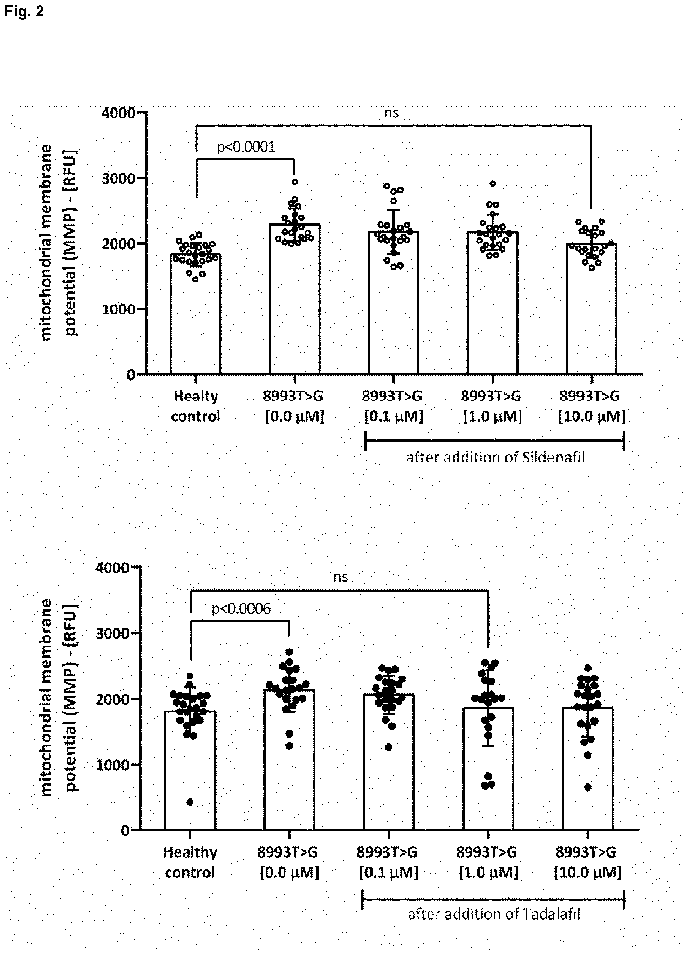

**Figure 2****: Mitochondrial membrane potentials (MMPs) in NPCs from MILS patients.** Measurement of mitochondrial membrane potentials (MMPs) in MILS patient-derived neural progenitor cells (NPCs) using the potentiometric fluorescent dye TMRM. Cells were exposed to increasing concentrations of the compounds sildenafil and tadalafil at concentrations ranging from 0.1 to 10.0 µM. Error bars denote the mean and standard deviation. Significance values were calculated using the nonparametric Man-Whitney test. The significance level was set at p<0.01. RFU, relative TMNM fluorescence units.

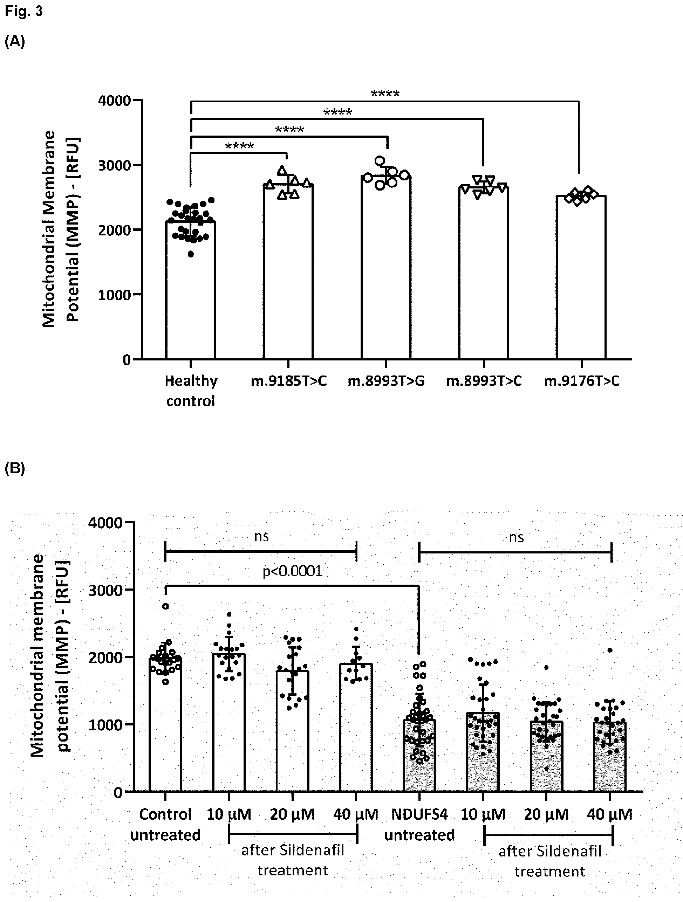

**Figure 3****: Mitochondrial membrane potentials (MMPs) in NPCs from MILS patients and Leigh syndrome patient with NDUFS4 mutation. (A)**Measurement of mitochondrial membrane potentials (MMPs) in NPCs derived from MILS patients with different MT-ATP6 mutations (mutation load = 100%). Regardless of mutation, a highly significant elevated MMP is found. Error bars denote the mean and standard deviation. (**B**) Measurement of mitochondrial membrane potentials (MMPs) in control NPC cells and in NPC cells from a patient with Leigh syndrome and a homozygous mutation in the nuclear-encoded NDUFS4 gene [NM_002495.4, c.462_462delA, p.(Lys154Asnfs*35)]. In contrast to Leigh patients with a mutation in the MT-ATP6 gene, those with inactivating NDUFS4 mutations show a decrease in MMP. Neither in controls nor in the patient with NDUFS4 mutation did the MMP change, even at high non-physiological sildenafil concentrations. Significance values were calculated using the non-parametric Man-Whitney test. The significance level was set at p<0.01. ****, p<0.0001. RFU, relative TMNM fluorescence units.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

Described in more detail below is:
- Investigating pathomechanisms of MILS and screening for compounds potentially effective in the treatment of MILS
- Evaluation of a dose-response curve for PDE5 inhibitors
- Validation of a PDE5 inhibitor therapeutic effect in different NPC cell lines carrying a mutation within the MT-ATP6 gene
- Patient data and demonstrated effects of PDE5 inhibitors in human patients suffering from MILS

### Example 1: Investigation of the PDE5 inhibitor class for MILS patients.

In one of our studies, we focused on maternally inherited Leigh syndrome (MILS), which is mostly caused by mutations in the mtDNA-encoded MT-ATP6 gene, which encodes a component of Complex V of the respiratory chain (F1F0-ATPase), where the actual ATP production of the cell takes place. For a long time it was unclear how these mutations act pathophysiologically.

We could show in a cell model of MILS that especially dopaminergic neurons (i.e. neurons that use the neurotransmitter dopamine as a transmitter) are preferentially damaged by mutations in the MT-ATP6 gene. As a cell model, we had chosen neuronal precursor cells (NPCs) differentiated from induced pluripotent stem cells (iPSCs) obtained from MILS patients. These NPC cells were characterized by both their mitochondrial membrane potential (MMP) being markedly elevated (hyperpolarization) and significantly reduced ATP production. These abnormalities were characteristic of the neurons, as they were not detectable in the patients' original skin fibroblasts. This explained for the first time the characteristic pattern of damage in the brains of MILS patients, which particularly affected dopaminergic neurons.

We discovered the actual neuron-damaging pathomechanism to be a disruption of the calcium signaling pathway, the components of which were consistently downregulated in patient neurons, leading to decreased intracellular calcium release. This was caused by hyperpolarization of mitochondria (i.e., an increase in mitochondrial membrane potential, MMPs), which was due to deficient function of F1F0-ATPase. Since the MMP can be optically read by potentiometric dyes (tetramethylrhodamine ethyl ester (TMRE), or tetramethylrhodamine methyl ester (TMRM) molecular probes), this was used to develop a fluorescent assay to perform automated high content screening (HCS), in which 130 compounds were screened. Drug therapies with which a favorable influence on the course of the syndromes described above could be demonstrated did not exist until now.

As shown in Figure 1, the PDE5 inhibitor avanafil was able to decrease the mitochondrial membrane potential of patient NPCs by more than 2 standard deviations.

Based on the finding that a PDE5 inhibitor (avanafil) can normalize the MMP of MILS patients, we tested (i) several relevant members of the compound class in a systematic manner (ii) on several different MILS patient cell lines (NPCs) covering the common mutations in the MT-ATP6 gene in humans. In particular, we focused on compounds that are already approved or tested for use as long-term therapeutics in children, such as sildenafil and tadalafil.

### Example 2: Confirmation of the therapeutic effect using a dose-response curve.

In vitro, a standard patient NPC cell line with a homoplasmic m.8993T>G mutation (mutation load = 100%) in the mtDNA was treated with sildenafil and tadalafil at increasing doses (0.1, 1.0, 10.0 µM) and the MMP was determined by a TMRM fluorescence assay (Figure 2). The lipophilic cation of the TMRM dye accumulated in the mitochondrial matrix in proportion to the level of MMP. Relative fluorescence units, RFU readout at the wavelength of 600 nm was performed. The strength of fluorescence emission correlated with the potential difference between mitochondrial matrix and cytosol. Increasing doses resulted in a gradual normalization of the MMP. Serum concentrations detected in adult patients one hour after a single dose of 25, 50, 100, and 200 mg sildenafil resulted in serum concentrations of 84 - 157 - 328 - 903 ng/ml (corresponding to 0.17 - 0.33 - 0.69 - 1.90 µM). When the steady-state plasma concentration of sildenafil was measured in patients with pulmonary arterial hypertension (PAH) after taking 3 x 80 mg/d (3 mg/kg/d), the peak level was 580 ± 45 ng/ml (0.28 ± 0.02 µM). When we measured the steady-state plasma concentration of tadalafil in patients with pulmonary arterial hypertension (PAH) after taking 10 - 40 mg/d, the peak level was 314 - 991 ng/ml (0.81 - 2.54 µM). This means that under the experimental conditions described above, we were in a range that is pharmacologically effective and safe.

Dose-dependent reduction of MMPs to normal values was found for two chemically and structurally very different molecules (sildenafil and tadalafil, see below), which have PDE5 inhibition in common.

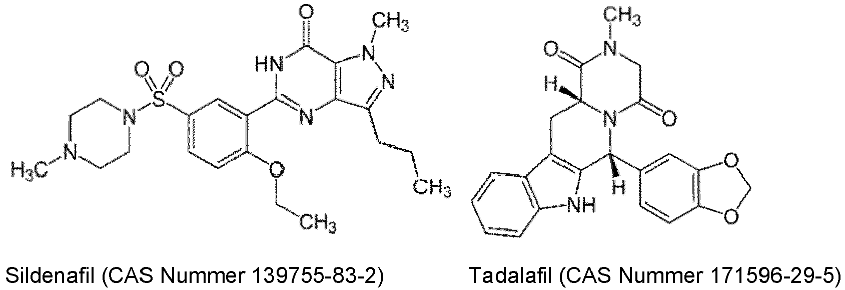

### Example 3: Validation of therapeutic effect in different NPC cell lines derived from patients with different MT-ATP6 mutation.

To validate the basal disease mechanism (and thus the efficacy of PDE5 inhibitors), we examined whether different NPCs derived from patients with different MT-ATP6 mutations all also exhibited elevation of MMPs (Figure 3A).

We could show that the effect of MMP increase is specific for mutations in MT-ATP6. Mutations in a nuclear encoded gene (SURF1), which is the most common cause of Leigh syndrome, did not increase MMP (Figure 3B). In conclusion, we suggest that therapy with PDE5 inhibitors is effective only in patients with the Leigh syndrome variant or a disease, which are caused by mutations in genes of the functional subunit of mitochondrial Complex V (F1F0-ATPase), as only these cells show an increase in MMP.

### Example 4: Individual therapy trials prove efficacy of PDE5 inhibitors.

Based on the study situation with numerous pharmacological safety and efficacy studies in children and neonates with pulmonary arterial hypertension (PAH) and with lymphatic malformations, we decided to perform an individual therapy trial with sildenafil in four patients with MILS, because no other therapies were available. Numerous studies already exist for sildenafil that have tested its use in adult pulmonary hypertension and in neonates. In adults, doses ranging from 3 x 20 mg to 3 x 80 mg have been used. Doses used in neonates ranged from 2-12 mg/kg/d.

Patient data of relevance (anonymized) are summarized in the following table:

| **Patient A** | |
|---|---|
| **Age, Gender** | 16 years, male |
| **Mutation** | MT-ATP6 m.9176T>G |
| **Symptoms** | Encephalopathy, cerebral seizures, severe leg-emphasized motor-sensory neuropathy, mechanical ventilation due to respiratory insufficiency, hardly any spontaneous movements possible, cardiomyopathy (LVEF around 45%, histology: chronic myocardial damage, macrophage-dominated inflammatory reaction), nutrition only possible via PEG feeding tube. |
| **Sildenafil Dosis** | 3 x 40 mg (long-term medication), 2 mg/kg KG/d |

| **Therapy progress** | |
|---|---|
| **After 1 month** | Extubation possible |
| **After 3 months** | Sitting stably, movements against gravity possible, stable respiration, ECHO cardiography LVEF 65% |
| **After 5 months** | Tracheostomy closure and PEG feeding tube removal, EEG normal |
| **After 10 months** | Back to regular school attendance |
| **After 18 months** | Independently moves with the wheelchair, even curbs and ascending terrain |
| **After 30 months** | Lifts himself into a wheelchair, walks at times with a walker with forearm supports. Medication was very well tolerated throughout the period. Cardiac checks have been normal the entire time. Cerebral seizures did not recur. |
| **After 4 years** | The patient can move independently in his wheelchair and move himself from the wheelchair to bed and back. With a walker (with forearm support), he can walk around the home. His clinical situation is stable and he is currently in vocational training to become an office clerk. No further metabolic crises did occur. |

| **Patient B** | |
|---|---|
| **Age, Gender** | 15 years, male |
| **Mutation** | MT-ATP6 m.8993T>G |
| **Symptoms** | Frequent metabolic derailments in the context of febrile infections (about 2 times per year), sometimes with focal neurologic symptoms (only slow recovery), free walking distance 500 m at maximum. |
| **Sildenafil Dosis** | 3 x 20 mg (long-term medication), 1.2 mg/kg KG/d |
| **Therapy progress** | |
| **After 2 months** | Increase in free walking distance from 500 m to >2000 m, significantly less exhaustible, increase in speed and safety in the Nine-Hole-Peg Test. No more metabolic derailment recorded to date. Medication is well tolerated, normal cardiac examination findings. |
| **After 3 years** | No further metabolic crisis have occurred, he can walk long distances and play tennis. The medication is still well tolerated, the cardiological examinations have always been normal. |

| **Patient C** | |
|---|---|
| **Age, Gender** | 9 months, male |
| **Mutation** | MT-ATP6 m.8570T>C |
| **Symptoms** | Floppy infant (severe muscle hypotonia), too weak for suckling, anaemia (5 g/dL), lactate elevation (5.48 mmol/L), hypertrophic cardiomyopathy. By 9 months of age, 10 inpatient stays were required due to metabolic crises associated with febrile infections. Palliative care was planned. |
| **Sildenafil Dosis** | 3 x 4 mg (long-term medication), 1.5 mg/kg KG/d |

| **Therapy progress** | |
|---|---|
| **After 12 months** | No more palliative care required. No metabolic derailment observed since starting treatment and has progressed in motor development: can turn, can swallow soft foods, can sit stably at table, head control exists, has gained weight well. The medication is well tolerated. |
| **After 3 years** | The patient's clinical condition is stable, no further metabolic crises occurred after the start of treatment. He suffered several febrile illnesses without metabolic crises. |

| **Patient D** | |
|---|---|
| **Age, Gender** | 21 years, female |
| **Mutation** | MT-ATP6 m.9185T>C |
| **Symptoms** | Relapsing episodes of muscle weakness (4-5 per week), severe demyelinating neuropathy, urinary incontinence, chronic metabolic acidosis, exercise insufficiency. |
| **Sildenafil Dosis** | 3 x 25 mg (long-term medication), 1.4 mg/kg KG/d |

| **Therapy progress** | |
|---|---|
| **After 4 months** | Significant decrease in paralytic episodes. The medication is well tolerated. |

### References:

1. Stendel C, Neuhofer C, Floride E, Yuqing S, Ganetzky RD, Park J, Freisinger P, Kornblum C, Kleinle S, Schöls L, Distelmaier F, Stettner GM, Büchner B, Falk MJ, Mayr JA, Synofzik M, Abicht A, Haack TB, Prokisch H, Wortmann SB, Murayama K, Fang F, Klopstock T. Delineating MT-ATP6-associated disease. Neurol Genet 2020;6. doi:10.1212/NXG.0000000000000393
2. Rahman S, Blok RB, Dahl HH, Danks DM, Kirby DM, Chow CW, Christodoulou J, Thorburn DR. Leigh syndrome: clinical features and biochemical and DNA abnormalities. Ann Neurol 1996;39:343-51.
3. Thorburn DR, Rahman J, Rahman S. Mitochondrial DNA-Associated Leigh Syndrome and NARP. In: Adam MP, Ardinger HH, Pagon RA, Wallace SE, Bean LJ, Mirzaa G, Amemiya A, eds. GeneReviews®. Seattle (WA): : Uni-versity of Washington, Seattle 1993. http://www.ncbi.nlm.nih.gov/books/NBK1173/ (accessed 2 Aug2021).
4. Van Maldergem L, Trijbels F, DiMauro S, Sindelar PJ, Musumeci O, Janssen A, Delberghe X, Martin JJ, Gillerot Y. Coenzyme Q-responsive Leigh's encephalopathy in two sisters. Ann Neurol. 2002 Dec;52(6):750-4. doi: 10.1002/ana.10371. PMID: 12447928.
5. US 2021/177846 A1 (HADCOCK JOHN R [US]) 17 June 2021
6. WO 01/10406 A2 (UNIV TEXAS [US]; SPONSEL WILLIAM E [US]) 15 February 2001
7. Mollá B, Muñoz-Lasso DC, Calap P, Fernandez-Vilata A, de la Iglesia-Vaya M, Pallardó FV, Moltó MD, Palau F, Gonzalez-Cabo P. Phosphodiesterase Inhibitors Revert Axonal Dystrophy in Friedreich's Ataxia Mouse Model. Neurotherapeutics. 2019 Apr;16(2):432-449. doi: 10.1007/s13311-018-00706-z. PMID: 30761510; PMCID: PMC6554462.
8. Percival JM, Whitehead NP, Adams ME, Adamo CM, Beavo JA, Froehner SC. Sildenafil reduces respiratory muscle weakness and fibrosis in the mdx mouse model of Duchenne muscular dystrophy. J Pathol. 2012 Sep;228(1):77-87.
9. Catteruccia M, Verrigni D, Martinelli D, Torraco A, Agovino T, Bonafé L, D'Amico A, Donati MA, Adorisio R, Santorelli FM, Carrozzo R, Bertini E, Dionisi-Vici C. Persistent pulmonary arterial hypertension in the newborn (PPHN): a frequent manifestation of TMEM70 defective patients. Mol Genet Metab. 2014 Mar;111(3):353-359.
10. Smith FM, Kosman DJ. Molecular Defects in Friedreich's Ataxia: Convergence of Oxidative Stress and Cytoskeletal Abnormalities. Front Mol Biosci. 2020 Nov 9;7:569293.
11. Vu Hong A, Sanson M, Richard I, Israeli D. A revised model for mitochondrial dysfunction in Duchenne muscular dystrophy. Eur J Transl Myol. 2021 Sep 17;31(3):10012.
12. Percival JM, Siegel MP, Knowels G, Marcinek DJ. Defects in mitochondrial localization and ATP synthesis in the mdx mouse model of Duchenne muscular dystrophy are not alleviated by PDE5 inhibition. Hum Mol Genet. 2013 Jan 1;22(1):153-67.

All references are incorporated herein in their entirety.

## Claims

1. A phosphodiesterase 5 (PDE5) inhibitor for use in the treatment and/or prevention of a medical condition associated with mitochondrial Complex V (ATP synthase) deficiency in a human subject.

2. The PDE5 inhibitor for use according to claim 1, wherein the mitochondrial Complex V deficiency comprises at least one DNA mutation in a structural subunit gene, or in an assembly gene, of the mitochondrial Complex V (ATP synthase).

3. The PDE5 inhibitor for use according to claim 2, wherein the DNA mutation occurs in the mitochondrial membrane subunit 6 gene (MT-ATP6).

4. The PDE5 inhibitor for use according to claim 2, wherein the DNA mutation is a nuclear mutation and is homozygous or compound heterozygous for a structural subunit or an assembly factor of the mitochondrial Complex V.

5. The PDE5 inhibitor for use according to claim 2 or 3, wherein the mitochondrial DNA mutation is homoplasmic or heteroplasmic, wherein said heteroplasmic mitochondrial DNA mutation is present in at least 30% of mitochondria per cell, preferably 60% or more, more preferably 70% or more.

6. The PDE5 inhibitor for use according to any of the preceding claims, wherein the mutation in the mitochondrial Complex V structural subunit or assembly gene comprises a variant at one or more nucleic acid positions selected from Table 2 in mitochondrial DNA or the nuclear genome.

7. The PDE5 inhibitor for use according to any of the preceding claims, wherein the medical condition comprises a Maternally Inherited Leigh syndrome (MILS), a Neuropathy, an Ataxia or a Retinitis Pigmentosa (NARP) or a neurological syndrome associated with a mitochondrial Complex V deficiency.

8. The PDE5 inhibitor for use according to any of the preceding claims, wherein the PDE-5 inhibitor is avanafil.

9. The PDE5 inhibitor for use according to any of the preceding claims, wherein the PDE-5 inhibitor is sildenafil.

10. The PDE5 inhibitor for use according to any of the preceding claims, wherein the PDE-5 inhibitor is tadalafil.

11. The PDE5 inhibitor for use according to any of the preceding claims, wherein the PDE-5 inhibitor is selected from the group consisting of vardenafil, mirodenafil, udenafil, and lodenafil.

12. The PDE5 inhibitor for use according to any of the preceding claims, wherein the inhibitor is administered:
a. At a dosage of 0.1-10 mg/kg/day to a human subject, preferably at 0.5-5 mg/kg/day, more preferably at 1-2 mg/kg/day, and/or
b. At a frequency of 2 to 4 times per day, preferably 4 times daily, wherein the route of administration is subcutaneously, intravenously or orally, preferably orally via a tablet of 1mg to 100mg per dose, more preferably 2mg to 80mg per dose, even more preferably 4mg to 40mg per dose.

13. The PDE5 inhibitor for use according to any of the preceding claims, wherein said dosage and frequency is configured to prevent and/or to reduce cGMP degradation to decrease intracellular calcium levels in striatal neuron mitochondria and/or to reduce mitochondrial membrane potential below a level at treatment initiation.

14. The PDE5 inhibitor for use according to any of the preceding claims, wherein the effect of said inhibitor to said subjects comprises:
- Preventing and/or inhibiting cGMP degradation and reducing mitochondrial membrane potential to levels comparable to healthy subjects,
- Reducing the number of paralytic events, motor and sensory neuropathy, metabolic crises events, lactic acidosis, cardiomyopathy, seizures, encephalopathy, stroke-like episodes, endocrine abnormalities, anemia, exercise insufficiency, muscle weakness, loss of muscle tone and/or muscular hypotonia, respiratory insufficiency, retinitis rigmentosa, ataxia, increased susceptibility to infections, developmental delay, intellectual disability, compared to treatment initiation, and/or
- Preventing and/or reducing the risk of said subject for repeated metabolic crises, palliative care or mortality, or resolves the need of the subject to receive palliative care.

15. A pharmaceutical composition comprising a PDE5 inhibitor for use as a medicament in the treatment and/or prevention of a medical condition according to any of the preceding claims, wherein said composition is in admixture with a pharmaceutically acceptable carrier and/or formulated in a pharmaceutically buffered solution.
